(19) Europäisches Patentamt
European Patent Office
Office européen des brevets

(11) Numéro de publication: **0 260 162 B1**

# (12) FASCICULE DE BREVET EUROPEEN

(45) Date de publication du fascicule du brevet: **14.11.90**

(51) Int. Cl.⁵: **C07C 65/36**, C07C 65/17, C07C 59/86, C07C 59/54

(21) Numéro de dépôt: **87401644.7**

(22) Date de dépôt: **10.07.87**

## (54) Dérivés bicycliques aromatiques, leur procédé de préparation et leur utilisation en médecine humaine et vétérinaire et en cosmétique.

(30) Priorité: **17.07.86 FR 8610423**

(43) Date de publication de la demande: **16.03.88 Bulletin 88/11**

(45) Mention de la délivrance du brevet: **14.11.90 Bulletin 90/46**

(84) Etats contractants désignés: **AT BE CH DE ES FR GB GR IT LI NL SE**

(56) Documents cités:
**EP-A- 0 123 535**
**US-A- 3 763 233**

(73) Titulaire: **CENTRE INTERNATIONAL DE RECHERCHES DERMATOLOGIQUES C.I.R.D. Groupement d'Intérêt Economique dit:, Sophia Antipolis, F-06560 Valbonne(FR)**

(72) Inventeur: **Maignan, Jean, 8, rue Halevy, F-93290 Tremblay les Gonesse(FR)**
Inventeur: **Lang, Gérard, 44, avenue Lacour, F-95210 Saint Gratien(FR)**
Inventeur: **Malle, Gérard, 18, Grande rue, F-77580 Villiers sur Morin(FR)**
Inventeur: **Restle, Serge, 140, rue Anatole France, F-93600 Aulnay-sous-Bois(FR)**
Inventeur: **Shroot, Braham Villa 35, Hameaux de Val Bosquet Chemin de Val-Bosquet, F-06600 Antibes(FR)**

(74) Mandataire: **Nony, Michel et al, 29, rue Cambacérès, F-75008 Paris(FR)**

**EP 0 260 162 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet de nouveaux dérivés bicycliques aromatiques, leur procédé de préparation et leur utilisation en médecine humaine et vétérinaire et en cosmétique.

Les composés selon l'invention présentent une activité dans le traitement topique et systémique des affections dermatologiques liées à un désordre de la kératinisation (différenciation-prolifération) et d'affections dermatologiques (ou autres) à composante inflammatoire et/ou immunoallergique et dans le traitement des maladies de dégénérescence du tissu conjonctif, ainsi qu'une activité anti-tumorale.

En outre ces composés peuvent être utilisés dans le traitement de l'atopie qu'elle soit cutanée ou respiratoire et du psoriasis rhumatoïde.

Ces composés présentent également une bonne activité sur les germes impliqués dans l'acné.

Enfin, ils trouvent une application dans le domaine ophtalmologique notamment dans le traitement des cornéopathies.

Les dérivés bicycliques aromatiques selon l'invention peuvent être représentés par la formule générale suivante:

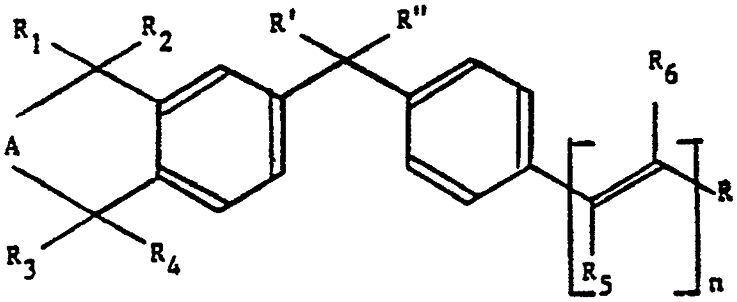

(I)

dans laquelle:

n est 0 ou 1,

R' représente un atome d'hydrogène, un radical OH, un radical alkoxy ayant de 1 à 4 atomes de carbone, ou un radical acyloxy ayant de 1 à 4 atomes de carbone,

R" représente un atome d'hydrogène ou un radical alkoxy ayant de 1 à 4 atomes de carbone,

ou R' et R", pris ensemble forment un radical oxo (=O), méthano (=$CH_2$) ou hydroxyimino (=N-OH),

R représente le radical -$CH_2OH$ ou le radical -$COR_7$,

$R_7$ représentant un atome d'hydrogène, le radical -$OR_8$ ou le radical

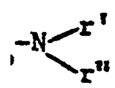

$R_8$ représentant un atome d'hydrogène, un radical alkyle ayant de 1 à 20 atomes de carbone, monohydroxyalkyle,

polyhydroxyalkyle, aryle ou aralkyle éventuellement substitué(s) ou un reste d'un sucre ou encore le radical

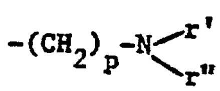

p étant 1, 2 ou 3 et r' et r" représentant un atome d'hydrogène, un radical alkyle inférieur, un radical monohydroxyalkyle éventuellement interrompu par un hétéroatome,

un radical polyhydroxyalkyle, un radical aryle ou benzyle éventuellement substitué(s), un reste d'aminoacide ou de sucre aminé ou pris ensemble forment avec l'atome d'azote auquel ils sont rattachés, un hétérocycle,

A représente un radical méthylène ou diméthylène, substitués ou non par un radical alkyle inférieur,

$R_1$, $R_2$, $R_3$ et $R_4$ représentent un atome d'hydrogène ou un radical alkyle inférieur,

$R_1$ et $R_3$ pris ensemble pouvant former un radical méthylène ou diméthylène, lorsque A représente un radical diméthylène,

$R_5$ et $R_6$ représentent un atome d'hydrogène ou un radical méthyle, et les sels desdits composés ainsi que leurs isomères optiques et géométriques.

Par radical alkyle inférieur on doit entendre un radical ayant de 1 à 6 atomes de carbone.

Parmi les radicaux alkyles inférieurs et ceux ayant jusqu'à 20 atomes de carbone on peut citer les radicaux méthyle, éthyle, isopropyle, butyle, tertiobutyle, ethyl-2 hexyle, isooctyle, dodécycle, hexadécyle et octadécyle.

Par radical monohydroxyalkyle on doit entendre un radical ayant 2 à 6 atomes de carbone notamment un radical hydroxy-2 éthyle, hydroxy-2 propyle ou hydroxy-2 éthoxyéthyle.

Par radical polyhydroxyalkyle, on doit entendre un radical contenant de 3 à 6 atomes de carbone et de 2 à 5 groupes hydroxyles tels que les radicaux dihydroxy-2,3 propyle, dihydroxy-1,3 propyl-2 ou le reste du pentaérythritol.

Comme radical alkoxy ayant de 1 à 4 atomes de carbone on peut citer le radical méthoxy, isopropoxy, butoxy ou tert-butoxy.

Par radical aryle, on doit entendre un radical phényle éventuellement substitué par au moins un atome d'halogène, -OH, $-NO_2$ , un radical alkyle inférieur, un radical trifluorométhyle ou une fonction acide carboxylique.

Comme radical aralkyle préféré on peut citer le radical benzyle ainsi que le radical phénéthyle.

Par reste d'un sucre, on doit entendre un reste dérivant par exemple du glucose, du mannose, de l'érythrose ou du galactose.

Parmi les restes de sucres aminés on peut citer ceux dérivant de glucosamine, de galactosamine, de mannosamine ou de la méglumine..

Lorsque les radicaux r' et r'' pris ensemble forment avec l'azote auquel ils sont rattachés un hétérocycle, celui-ci est de préférence un radical pipéridino, pipérazino, morpholino, pyrrolidino ou (hydroxy-2 éthyl)-4 pipérazino.

Quand les composés selon l'invention se présentent sous forme de sels, il peut s'agir soit de sels d'un métal alcalin ou alcalino-terreux ou encore de zinc, ou d'une amine organique lorsqu'ils comportent au moins une fonction acide libre, soit de sels d'un acide minéral ou organique notamment de chlorhydrate, de bromhydrate ou de citrate lorsqu'ils comportent au moins une fonction amine.

En fonction de la formule (I) ci-dessus, les composés de la présente demande peuvent être soit des dérivés bicycliques benzoïques de formule II soit des dérivés bicycliques cinnamiques de formule III:

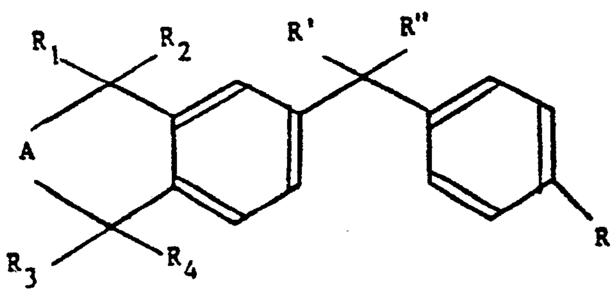

(II)

dans laquelle:
A, R , $R_1$à $R_4$ , R' et R'' ont les mêmes significations que celles données ci-dessus pour la formule I.

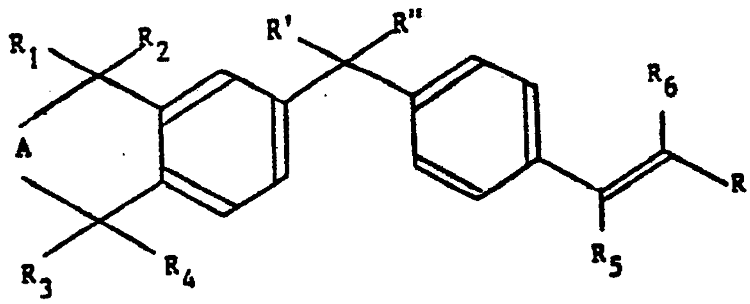

(III)

dans laquelle:
A, R, $R_1$ à $R_6$ , R' et R'' ont les mêmes significations que celles données pour la formule I.

Parmi les composés des formules II et III particulièrement préférés selon l'invention, on peut citer ceux correspondant aux formules IV et V suivantes:

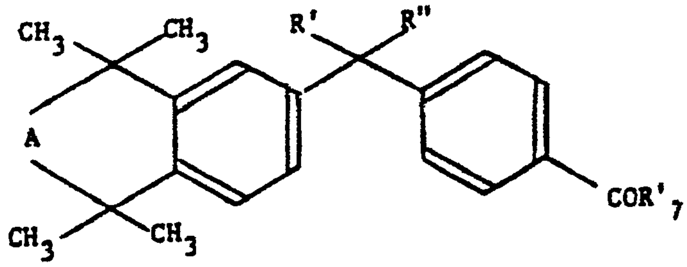

(IV)

dans laquelle:
A représente le radical

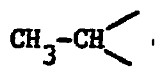

ou le radical diméthylène,
R′ représente un radical OH et R″ représente un atome d'hydrogène ou R′ et R″, pris ensemble, forment un radical oxo (=O),
et $R'_7$ représente un atome d'hydrogène, le radical $-OR'_8$ ou le radical

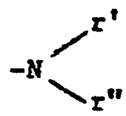

$R'_8$ représentant un atome d'hydrogène ou un radical alkyle inférieur,
r′ représentant un atome d'hydrogène et r″ représentant un radical alkyle inférieur ou un radical phényle substitué ou r′ et r″ pris ensemble forment un radical (hydroxy-2 éthyl)-4 pipérazino..

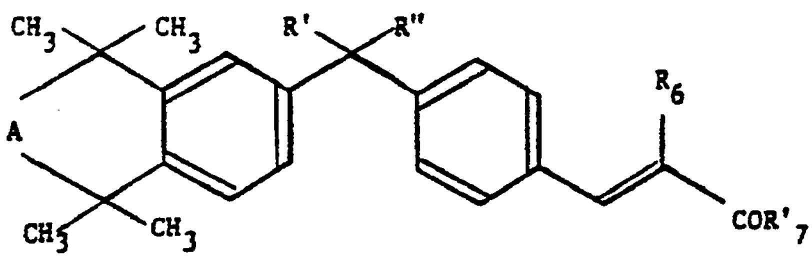

(V)

dans laquelle:
A représente le radical

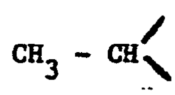

ou le radical diméthylène,
R′ représente un radical OH et R″ un atome d'hydrogène ou R′ et R″, pris ensemble, forment un radical oxo (=O),
$R_6$ représente un atome hydrogène ou un radical méthyle, et $R'_7$ représente $-OR'_8$ ou le radical

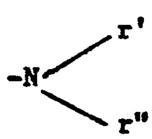

$R'_8$ représentant un atome d'hydrogène ou un radical alkyle inférieur,
r′ représentant un atome d'hydrogène et r″ représentant un radical alkyle inférieur.

Parmi les composés de formule (I) selon l'invention on peut notamment citer:

(1) le (tétraméthyl-5,5,8,8 tétrahydro-5,6,7,8 naphtyl-2) carbonyl-4 benzoate de méthyle,
(2) l'acide (tétraméthyl-5,5,8,8 tétrahydro-5,6,7,8 naphtyl-2) carbonyl-4 benzoïque,
(3) le (méthano-5,8 tétrahydro-5,6,7,8 naphtyl-2) carbonyl-4 benzaldéhyde,
(4) le (tétraméthyl-5,5,8,8 tétrahydro-5,6,7,8 naphtyl-2) carbonyl-4 benzaldéhyde,
(5) le (pentaméthyl-1,1,2,3,3 indanyl-5) carbonyl-4 benzoate de méthyle,
(6) l'acide (pentaméthyl-1,1,2,3,3 indanyl-5) carbonyl-4 benzoïque,
(7) le (pentaméthyl-1,1,2,3,3 indanyl-5) carbonyl-4 benzaldéhyde,
(8) le (pentaméthyl-1,1,2,3,3 indanyl-5)-1 (hydroxyméthyl-4 phényl)-1 méthanol,
(9) l'acide (tétraméthyl-5,5,8,8 tétrahydro-5,6,7,8 naphtyl-2) hydroxyméthyl-4 benzoïque,
(10) l'acide (pentaméthyl-1,1,2,3,3 indanyl-5) hydroxyméthyl-4 benzoïque,
(11) le (pentaméthyl-1,1,2,3,3 indanyl-5) hydroxyméthyl-4 benzoate de méthyle,
(12) le N-éthyl (tétraméthyl-5,5,8,8 tétrahydro-5,6,7,8 naphtyl-2) carbonyl-4 benzamide,
(13) le N-éthyl (pentaméthyl-1,1,2,3,3 indanyl-5) carbonyl-4 benzamide.
(14) le trans (méthano-5,8 tétrahydro-5,6,7,8 naphtyl-2) carbonyl-4 cinnamate d'éthyle,
(15) l'acide trans (méthano-5,8 tétrahydro-5,6,7,8 naphtyl-2) carbonyl-4 cinnamique,
(16) le trans (tétraméthyl-5,5,8,8 tétrahydro-5,6,7,8 naphtyl-2) carbonyl-4 cinnamate d'éthyle,
(17) l'acide trans (tétraméthyl-5,5,8,8 tétrahydro-5,6,7,8 naphtyl-2) carbonyl-4 cinnamique,
(18) le trans (tétraméthyl-5,5,8,8 tétrahydro-5,6,7,8 naphtyl-2) carbonyl-4 α-méthyl cinnamate d'éthyle,
(19) l'acide trans (tétraméthyl-5,5,8,8 tétrahydro-5,6,7,8 naphtyl-2) carbonyl-4 α-méthyl cinnamique,
(20) le trans (pentaméthyl-1,1,2,3,3 indanyl-5) carbonyl-4 α-méthyl cinnamate d'éthyle,
(21) l'acide trans (pentaméthyl-1,1,2,3,3 indanyl-5) carbonyl-4 α-méthyl cinnamique,
(22) le trans (tétraméthyl-5,5,8,8 tétrahydro-5,6,7,8 naphtyl-2) hydroxyméthyl-4 α-méthyl cinnamate d'éthyle,
(23) l'acide trans (tétraméthyl-5,5,8,8 tétrahydro-5,6,7,8 naphtyl-2) hydroxyméthyl-4 α-méthyl cinnamique,
(24) le N-éthyl trans (tétraméthyl-5,5,8,8 tétrahydro-5,6,7,8 naphtyl-2) hydroxyméthyl-4 α-méthyl cinnamide,
(25) le N-éthyl trans (tétraméthyl-5,5,8,8 tétrahydro-5,6,7,8 naphtyl-2) carbonyl-4 α-méthyl cinnamide,
(26) le N-éthyl trans (pentaméthyl-1,1,2,3,3 indanyl-5) carbonyl-4 α-méthyl cinnamide.
(27) le trans (tétraméthyl-5,5,8,8 tétrahydro-5,6,7,8 naphtyl-2) hydroxyméthyl-4 cinnamate d'éthyle,
(28) l'acide trans (tétraméthyl-5,5,8,8 tétrahydro-5,6,7,8 naphtyl-2) hydroxyméthyl-4 cinnamique,
(29) l'(hydroxy-2 éthyl)-4' pipérazino (tétraméthyl-5,5,8,8 tétrahydro-5,6,7,8 naphtyl-2) carbonyl-4 benzamide,
(30) le N-(ditrifluorométhyl-3,5 phényl-1) (tétraméthyl-5,5,8,8 tétrahydro-5,6,7,8 naphtyl-2) carbonyl-4 benzamide.

La présente invention a également pour objet le procédé de préparation des composés de formule (I).

Ces composés peuvent être obtenus selon différentes méthodes en fonction de leur structure. Les composés de formule (II) peuvent par exemple être avantageusement préparés selon le schéma réactionnel suivant:

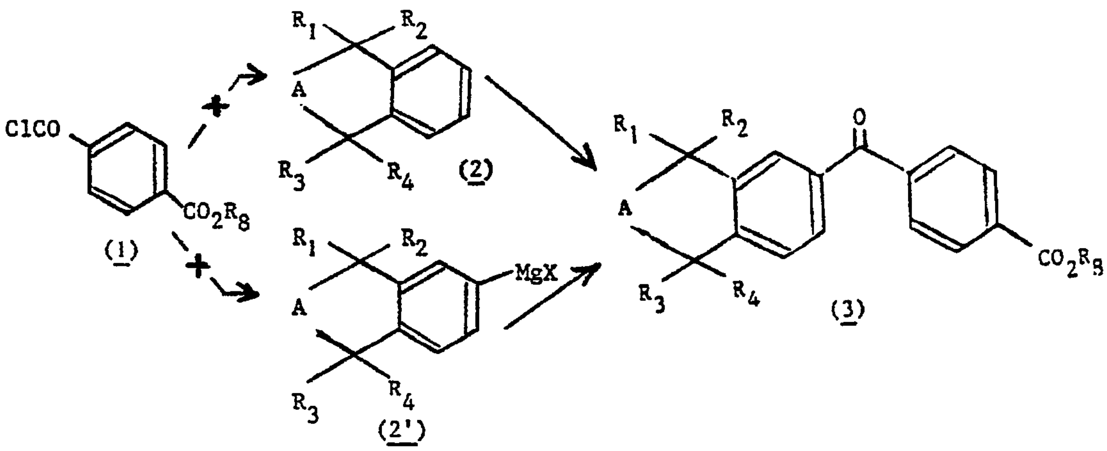

(1) (2) (2') (3)

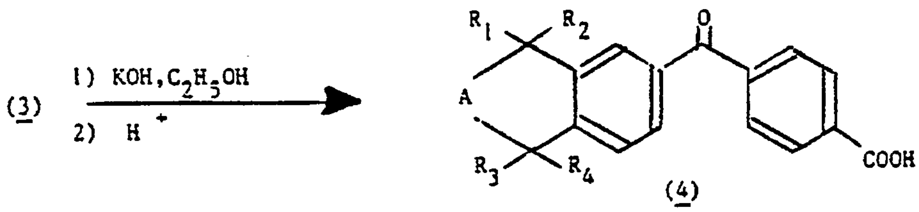


(3) (4)

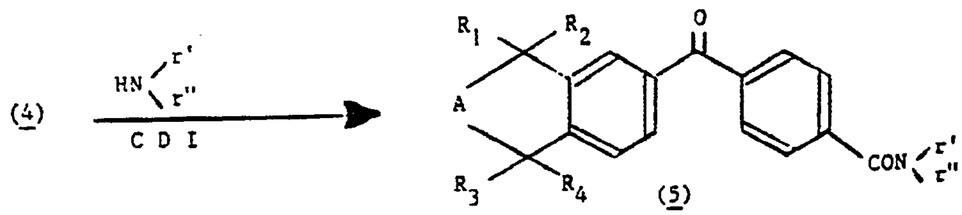


(4) (5)

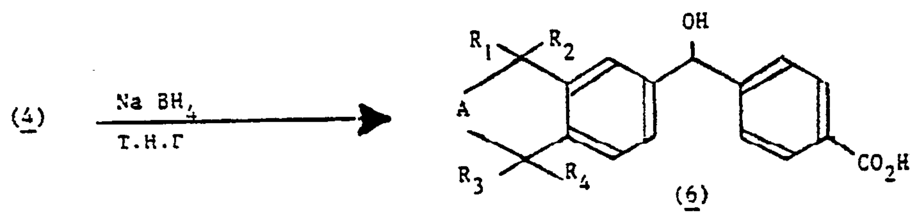


(4) (6)

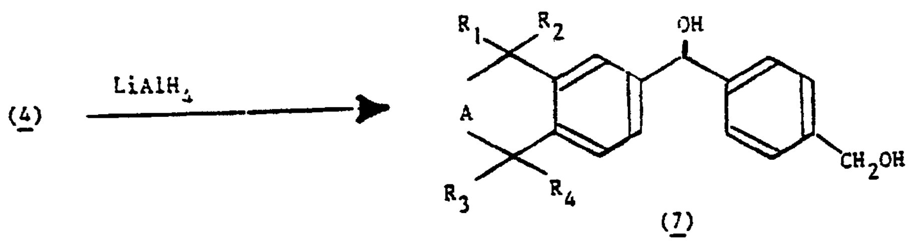


(4) (7)

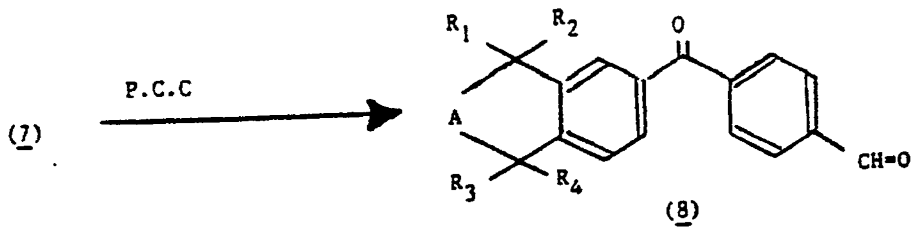


(7) (8)

$R_8 \neq H$
X = Br ou Cl

Le chlorure d'acide alkoxycarbonyl-4 benzoïque-2 (1) est obtenu au départ d'un paraformyl benzoate d'alkyle qui est oxydé en acide correspondant à l'aide d'un réactif de Jones puis transformé en chlorure d'acide par action de chlorure de thionyle selon la méthode classique de préparation des chlorures d'acide.

Parmi les produits de départ de formule (2) la tétraline et l'indane sont des produits commerciaux. Le tétraméthyl-5,5,8,8 tétrahydro-5,6,7,8 naphtalène (ou tétraméthyl-5,5,8,8 tétraline) est préparé suivant la méthode décrite par H.Q. BRUSON ET J.W. KROGER, J. Am. Chem. Soc-, 62, 36-44 (1940). Le méthano-5,8 tétrahydro-5,6,7,8 naphtalàne est obtenu selon la méthode décrite dans J. Org. Chem. 32, 893-901 (1967). Le pentaméthyl-1,1,2,3,3 indane et le tétraméthyl-1,1,3,3 indane sont obtenus selon les méthodes décrites dans le brevet français 1.392.804.

La réaction de condensation du chlorure d'acide alkoxycarbonyl-4 benzoïque-2 (1) sur le composé aromatique bicyclique (2) est effectuée dans les conditions habituelles de la réaction de Friedel-Crafts, c'est-à-dire en présence de chlorure d'aluminium ou de chlorure stanneux anhydre dans du dichloro-1,2 éthane à une température comprise entre 0° et 25°C sous agitation.

Le céto-ester (3) peut également être obtenu par réaction de condensation du chlorure d'acide alkoxycarbonyl-4 benzoïque-2 (1) sur le magnésien du dérivé halogéno aromatique (2') ce dernier étant obtenu dans le THF anhydre au reflux et la condensation étant réalisée à une température d'environ 0°C dans le mème solvant.

A partir du céto-ester (3) on accède par saponification au céto-acide correspondant (4) qui peut ensuite être transformé en amide de formule (5) par action d'une amine de formule

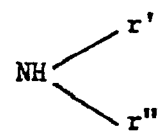

(r' et r" ayant les mêmes significations que données ci-dessus), en présence de N,N'-carbonyl diimidazole (CDI).

Lorsque $R_8$ représente un radical monohydroxy ou polyhydroxy-alkyle, il est préférable de préparer le céto-acide (4) à partir de l'ester méthylique (3) ($R_8$=-$CH_3$) et ensuite d'estérifier le céto-acide ainsi obtenu en céto-ester de l'alcool mono ou polyhydrique choisi selon les méthodes connues.

A partir du céto-acide (4) la réduction par le borohydrure de sodium dans un solvant organique tel que le T.H.F. permet d'obtenir l'alcool secondaire (6) et la réduction par l'hydrure de lithium aluminium du céto-acide (4) permet d'accéder au diol (7).

Par oxydation du diol (7) par le chlorochromate de pyridinium (PCC) on accède au céto-aldéhyde (8).

Le céto-aldéhyde (8) qui constitue également un intermédiaire pour la préparation des composés de formule (III) peut être également obtenu selon le schéma réactionnel suivant:

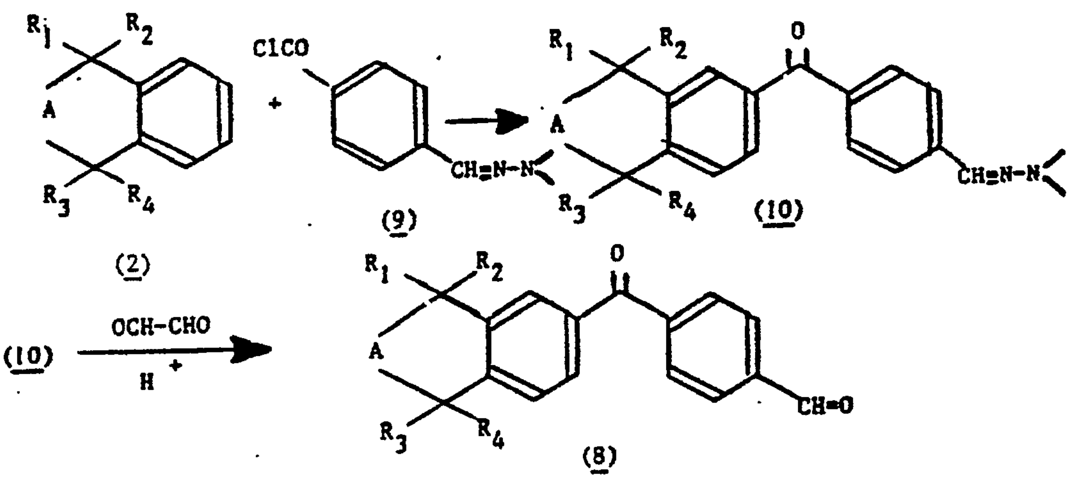

(2) (9) (10)

(10) (8)

Cette méthode consiste à réaliser une réaction de condensation du chlorure d'acide (9), dont la fonction aldéhyde a été préalablement protégée par formation d'une diméthylhydrazone, sur le composé aromatique bicyclique (1) dans les mêmes conditions que celles décrites ci-dessus pour la condensation du chlorure d'acide (2).

La fonction aldéhyde de la benzophénone obtenue (10) est ensuite libérée par échange avec le glyoxal pour conduire avec un bon rendement au céto-aldéhyde (8).

Les céto-aldéhydes (8) sont des produits de dépar particulièrement utiles pour la synthèse des composés de formule (III) selon la schéma réactionnel suivant:

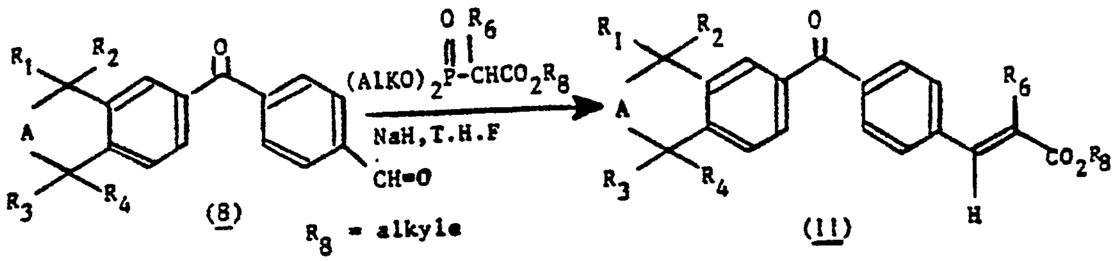


(8) (11)

La réaction de Wittig-Horner du céto-aldéhyde (8) avec le phosphonoacétate, substitué ou non, est réalisée en présence d'hydrure de sodium dans un solvant organique tel que le T.H.F.

Le céto-ester insaturé (11) obtenu peut ensuite être transformé comme précédemment en acide correspondant puis en amide par action d'une amine de formule

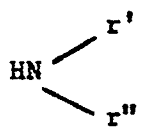
.

Les hydroxy-acides de formule (6) et hydroxy-esters (6') des composés de formule (III) peuvent être obtenus par réaction d'un organomagnésien préparé à partir du dérivé bromé (12) sur un formyl-4 cinnamate d'alkyle (13) selon le schéma réactionnel suivant:

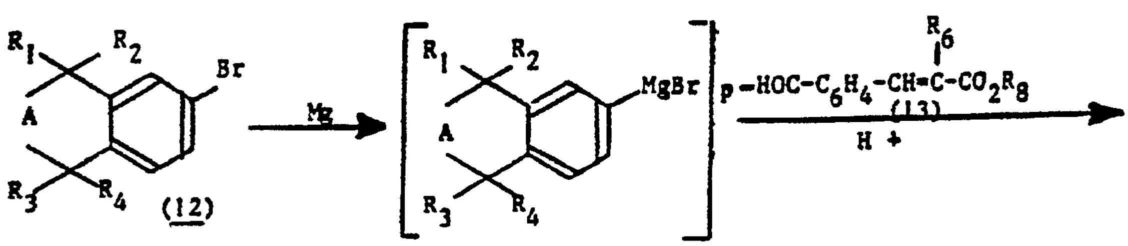


(12)

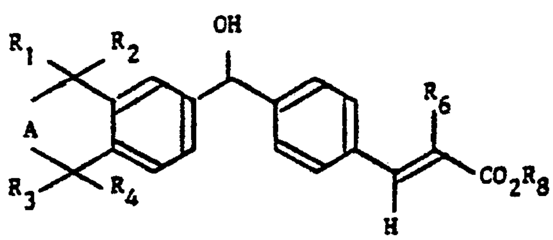

(6 , $R_8$=H)
(6', $R_8$ = alkyle)

Les formyl cinnamates d'alkyle (13) sont obtenus à partir du téréphtalaldéhyde (14) commercial, une des fonctions aldéhydes étant protégée sous forme de diméthylhydrazone. L'aldéhyde (15) ainsi obtenu est ensuite condensé sur un phosphonoacétate d'alkyle dans les conditions de la réaction de Wittig-Horner et la fonction aldéhyde protégée est alors libérée en milieu acide par échange avec le glyoxal pour obtenir le formyl cinnamate d'alkyle (13).

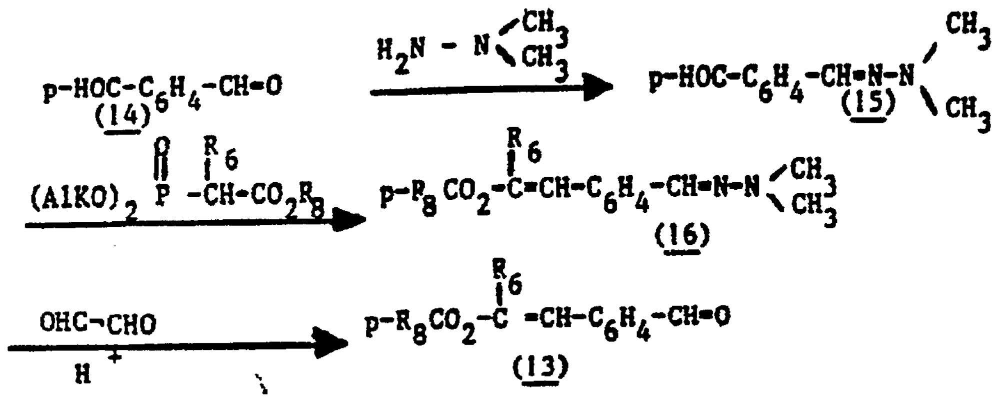


Les composés selon l'invention dans lesquels R'=R"=H sont obtenus par réduction au zinc des dérivés cétoniques dans l'acide acétique en présence d'acide chlorhydrique.

Les réactions de réduction du carbonyle doivent bien entendu être compatibles avec la nature du groupement R. Il peut être souhaitable d'en assurer la protection éventuelle, toutefois la réduction du carbonyle ne soulève aucune difficulté lorsque R=$CO_2H$.

Les dérivés acyloxy des composés de formule (I) (R'=acyloxy $C_1$-$C_4$ et R"=H) sont obtenus en faisant réagir une forme activée d'acide, tel qu'un anhydride ou un chlorure d'acide sur un composé selon l'invention dans lequel R'=OH et R"=H.

Les dérivés alkoxy des composés de formule (I) (R'=alkoxy $C_1$-$C_4$ et R"=H) sont de même obtenus à partir des composés de formule (I) (R'=OH et R"=H) selon les méthodes connues.

Pour la préparation des dérivés acyloxy et alkoxy il est préférable que le radical R soit une fonction ester, acide ou amide.

Les composés de formule (I) dans laquelle R' et R"=méthano ($CH_2$=) sont obtenus par réaction de Wittig selon le schéma réactionnel suivant:

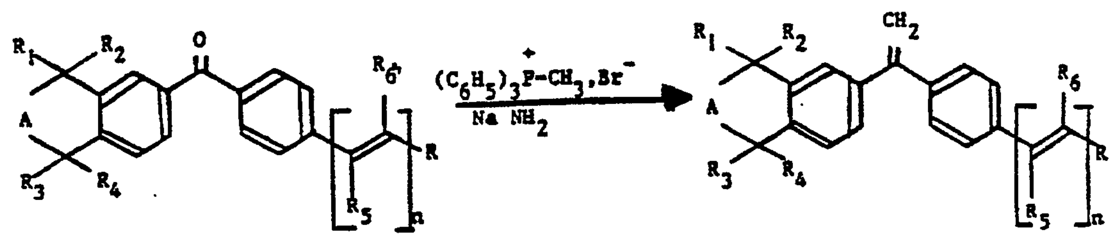


R ≠ CHO

Les composés de formule (I) dans laquelle R' et R"=hydroxyimino (=N-OH) sont obtenus par action de l'hydroxylamine sur les composés carbonylés correspondants.

La présente invention a également pour objet, à titre de médicament, les composés de formule (I) telle que définie ci-dessus.

Ces composés sont actifs dans le test d'inhibition de l'ornithine décarboxylase après induction, par "tape stripping", chez le rat nu [M. Bouclier et al, Dermatologica 169 n°4 (1984)]. Ce test est admis comme mesure d'une action antiproliférative.

Ces composés conviennent particulièrement bien pour traiter les affections dermatologiques liées à un désordre de la kératinisation (différenciation-prolifération) ainsi que les affections dermatologiques, ou autres, à composante inflammatoire et/ou immunoallergique notamment:

– les acnés vulgaires, comédoniennes ou polymorphes, les acnés séniles, solaires, et les acnés médicamenteuses ou professionnelles,
– les formes étendues et/ou sévères de psoriasis, et les autres troubles de la kératinisation, et notamment les ichtyoses et états ichtyosiformes,
– la maladie de Darier,
– les kératodermies palmo-plantaires,
– les leucoplasies et états leucoplasiformes, le lichen plan,
– toutes proliférations dermatologiques bénignes ou malignes, sévères ou étendues.

Ils sont également actifs dans le traitement des tumeurs, du psoriasis rhumatoïde, des atopies cutanées ou respiratoires ainsi que de certains problèmes ophtalmologiques relatifs aux cornéopathies.

Ces composés présentent également une bonne activité sur les germes impliqués dans l'acné.

Ainsi les composés selon l'invention présentent une bonne activité comédolytique dans le test sur la souris Rhino décrit par BONNE et al dans International Journal of Cosmetic Science 3, 23–28 (1981). Cette expérimentation est effectuée sur la peau de souris hairless rhino, préconisée comme modèle de screening d'agents comédolytiques par VAN SCOTT en 1972.

La présente invention a donc également pour objet des compositions médicamenteuses contenant au moins un composé de formule (I) tel que défini ci-dessus, ou un de ses sels, ou un de ses isomères optiques ou géométriques.

La présente invention a donc aussi pour objet une nouvelle composition médicamenteuse, destinée notamment au traitement des affections susmentionnées, caractérisée par le fait qu'elle comporte, dans un support pharmaceutique acceptable, au moins un composé de formule (I) et/ou un de ses sels et/ou un de ses isomères optiques ou géométriques.

Les composés selon l'invention sont généralement administrés à une dose journalière d'environ 2 µg/kg à 2 mg/kg de poids corporel.

Comme support des compositions, on peut utiliser tout support conventionnel, le composé actif se trouvant soit à l'état dissous, soit à l'état dispersé dans le véhicule.

L'administration peut être effectuée par voie entérale, parentérale, topique ou oculaire. Par voie entérale, les médicaments peuvent se présenter sous forme de comprimés, de gélules, de dragées, de sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions pharmaceutiques à base des composés selon l'invention se présentent sous forme d'onguents, de teintures, de crèmes, de pommades, de poudres, de timbres, de tampons imbibés, de solutions, de lotions, de gels, de sprays ou encore de suspensions.

Ces compositions par voie topique peuvent se présenter soit sous forme anhydre, soit sous forme aqueuse selon l'indication clinique.

Par voie oculaire, ce sont particulièrement des collyres.

Les compositions par voie topique ou oculaire contiennent de préférence de 0,0005 à environ 5% en poids d'au moins un composé de formule (I) elle que définie ci-dessus, par rapport au poids total de la composition.

Les composés de formule (I) selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la prévention ou le traitement des effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels et/ou un de ses isomères, cette composition se présentant notamment sous forme de lotion, gel, crème, savon ou shampooing.

La concentration en composé de formule (I), dans les compositions cosmétiques, est comprise entre 0,0005 et 2% en poids et de préférence entre 0,01 et 1% en poids.

Les compositions médicamenteuses et cosmétiques, selon l'invention, peuvent contenir des additifs inertes ou même pharmacodynamiquement ou cosmétiquement actifs et notamment: des agents hydratants, comme la thiamorpholinone et ses dérivés ou l'urée; des agents antiséborrhéiques ou antiacnéiques, tels que la S-carboxyméthylcystéine, la S-benzylcystéamine, leurs sels et leurs dérivés, la tioxolone ou le peroxyde de benzoyle; des antibiotiques comme l'érythromycine et ses esters, la néomycine, les tétracyclines et les polyméthylène-4,5 isothiazolones-3; des agents favorisant la repousse des cheveux, comme le "Minoxidil" (diamino-2,4 pipéridino-6 pyrimidine oxyde-3) et ses dérivés, le Diazoxide (chloro-7 méthyl-3 benzothiadiazine-1,2,4 dioxyde-1,1) et le Phénytoïn (diphényl-5,5 imidazolidine dione-2,4); des agents anti-inflammatoires stéroïdiens et non stéroïdiens; des caroténoïdes et, notamment le β-carotène; des agents anti-psoriasiques tels que l'anthraline et ses dérivés et les acides eicosatétraynoïque-5,8,11,14 et triynoïque-5,8,11 ainsi que leurs esters et amides; des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des anti-oxydants tels que de l'α-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

EXEMPLE I

Préparation du (tétraméthyl-5,5,8,8 tétrahydro-5,6,7,8 naphtyl-2) carbonyl-4 benzoate de méthyle.

Composé de formule IV dans laquelle A=$-(CH_2)_2-$, R′ et R″=oxo et $R'_7$=$-OCH_3$.

a) Préparation de l'acide méthoxycarbonyl-4 benzoïque.

A une solution de 20g de formyl-4 benzoate de méthyle dans 150cm3 d'acétone on ajoute, goutte à goutte, une solution contenant 30g de bichromate de potassium, dans 150cm3 d'eau et 27cm3 d'acide sulfurique concentré. On maintient l'agitation pendant deux heures à la température ordinaire. Après évaporation de l'acétone sous pression réduite, le mélange réactionnel est extrait à l'acétate d'éthyle.

La phase organique est séchée sur sulfate de magnésium puis concentrée. On obtient 11g d'acide méthoxycarbonyl-4 benzoïque brut que l'on recristallise dans l'acétate d'éthyle. Les cristaux sont essorés et séchés. Point de fusion :222°C. Le spectre de $^1H$ R.M.N correspond à la structure attendue.

b) Préparation du chlorure de l'acide méthoxycarbonyl-4 benzoïque.

Une suspension de 5g de l'acide obtenu en (a) ci-dessus dans 50cm3 de chlorure de thionyle est portée pendant trois heures à 40°C. A la fin de la réaction, le milieu est homogène et la solution est concentrée sous pression réduite. Le chlorure d'acide attendu, cristallise sous forme de paillettes roses. Le rendement en chlorure d'acide est quantitatif et ce dernier est directement utilisé pour l'étape suivante.

c) Préparation du (tétraméthyl-5,5,8,8 tétrahydro-5,6,7,8 naphtyl-2) carbonyl-4 benzoate de méthyle.

A une solution agitée à une température de 5°C, de 4,85g (0,0258 mole) de tétraméthyl-5,5,8,8 tétrahydro-5,6,7,8 naphtalène et de 5,4g (0,0272 mole) du chlorure d'acide obtenu en (b) ci-dessus dans 250cm3 de dichloro-1,2-éthane anhydre, on ajoute par petites portions, 5,4g de chlorure d'aluminium. L'agitation est maintenue pendant une demi-heure après la fin de l'addition et le milieu réactionnel est ensuite abandonné une nuit à la température ordinaire. Après avoir versé sur de la glace, la phase organique est décantée et la phase aqueuse est extraite au dichlorométhane.

Les phases organiques sont rassemblées puis lavées à l'aide d'une solution saturée de chlorure d'ammonium et séchées sur sulfate de magnésium.

Ces solutions sont concentrées puis déposées sur une colonne de chromatographie de gel de silice. Le produit attendu est élué au mélange hexane-acétate d'éthyle 9-1.

Après évaporation des phases d'élution, on obtient 3g de produit blanc que l'on recristallise dans l'hexane. Les cristaux sont essorés et séchés. Le (tétraméthyl-5,5,8,8 tétrahydro-5,6,7,8 naphtyl-2) carbonyl-4 benzoate de méthyle a un point de fusion de 136°C.

Analyse élémentaire: $C_{23}H_{26}O_3$, 1/4 $H_2O$

Calculé: C: 77,82 H: 7,38 O: 14,65

Trouvé: 77,53 7,42 14,78

EXEMPLE II

Préparation de l'acide (tétraméthyl-5,5,8,8 tétrahydro-5,6,7,8 naphtyl-2) carbonyl-4 benzoïque.

Composé de formule IV dans laquelle A=$-(CH_2)_2-$ , R′ et R″=oxo, et $R'_7$=$-OH$

Un mélange de 1,5g de l'ester obtenu à l'exemple I dans 100cm3 d'éthanol et 100cm3 de potasse 6N est porté sous agitation pendant deux heures à 50°C. L'éthanol est ensuite éliminé par évaporation sous vide. La phase aqueuse obtenue est alors acidifiée par addition d'acide chlorhydrique concentré. L'acide attendu précipite et est essoré, puis séché et recristallisé dans un mélange toluène-hexane. On isole ainsi 0,87g d'acide (tétraméthyl-5,5,8,8 tétrahydro-5,6,7,8 naphtyl-2) carbonyl-4 benzoïque de point de fusion: 193°C.

Le spectre $^1H$ R.M.N 250 MHz correspond à la structure attendue.

Analyse élémentaire: $C_{22}H_{24}O_3$

Calculé: C: 78,54 H: 7,19 O: 14,27

Trouvé: 78,50 7,23 14,20

EXEMPLE III

Préparation du (méthano-5,8 tétrahydro-5,6,7,8 naphtyl-2) carbonyl-4 benzaldéhyde.

Composé de formule II dans laquelle A=$-(CH_2)_2-$ ,$R_1$et $R_3$=$-CH_2-$ , $R_2$=$R_4$=H, R' et R"=oxo et R=-CHO.

a) Préparation du chlorure de l'acide paraformylbenzoïque dont la fonction aldéhyde est protégée sous forme de diméthylhydrazone.

(i) Préparation de l'acide formyl-4 benzoïque N,N-diméthyl-hydrazone.

A une solution agitée à une température de 30°C, de 49,25g (0,30 mole) de formyl-4 benzoate de méthyle dans 200cm3 de T.H.F anhydre, on ajoute goutte à goutte 22,8cm3 de N,N-diméthylhydrazine. L'agitation est maintenue une heure après la fin de l'addition et le solvant est rectifié sous pression réduite.

On obtient 61,15g d'un produit cristallisé jaune dont le spectre de $^1H$ R.M.N correspond à la structure attendue.

Ce solide est alors solubilisé dans un mélange de 200cm3 d'éthanol et de 200cm3 de potasse aqueuse 6N porté à 60°C.

Après 1h30 d'agitation à cette température, l'éthanol est évaporé. La phase aqueuse est diluée dans 1 litre d'eau et la solution obtenue est acidifiée par addition d'acide acétique. L'acide formyl-4 benzoïque N,N-diméthylhydrazone précipite. Il est essoré puis séché et on obtient 44,16g de cristaux jaunes de point de fusion:61°C. Le spectre $^1H$ R.M.N correspond à la structure attendue.

(ii) Préparation du chlorure d'acide.

A une suspension de 20g (0,10 mole) de l'acide obtenu ci-dessus dans 300cm3 de diéthyloxyde, on ajoute, sous agitation, 24,85cm3 de dicyclohexylamine. Après une heure le sel de dicyclohexylamine est essoré puis séché. On obtient ainsi 44,7g d'un sel de couleur crème de point de fusion :164°C. A une suspension de 11,5g (0,0308 mole) de ce sel dans 250cm3 de dichloro-1,2 éthane, on ajoute, goutte à goutte 6cm3 de chlorure de thionyle. L'agitation est ensuite maintenue une heure puis le précipité de chlorure de dicyclohexylammonium est filtré. Par évaporation du filtrat sous pression réduite, on isole 5,8g d'une poudre brune dont le spectre $^1H$ R.M.N correspond à la structure attendue. Le chlorure de l'acide paraformyl-4 benzoïque N,N-diméthyl-hydrazone est utilisé sans autre purification pour la suite de la synthèse.

b) Préparation du (méthano-5,8 tétrahydro-5,6,7,8 naphtyl-2) carbonyl-4 benzaldéhyde N,N-diméthylhydrazone.

A un mélange agité à une température inférieure à 5°C de 3,1g de benzonorbornène dans 100cm3 de dichloro-1,2 éthane anhydre, on ajoute 4,3g (0,020 mole) de chlorure d'acide obtenu ci-dessus, puis, par petites portions, 4,3g de chlorure d'aluminium. A la fin de l'addition, le mélange est encore agité pendant deux heures, puis versé sur de l'eau glacée. La phase de dichloro éthane est décantée, séchée sur sulfate de magnésium et évaporée sous pression réduite. Le produit obtenu est extrait à l'acétate d'éthyle puis la phase organique est lavée à l'aide d'une solution aqueuse d'hydrogénocarbonate de sodium, et à l'eau.

Après séchage sur sulfate de magnesium, la solution d'acétate d'éthyle est concentrée, puis déposée sur une colonne de chromatographie de gel de silice et le produit attendu élué au mélange hexane-acétate d'éthyle (8-2). Après concentration des phases éluantes on obtient 2,6g de (méthano-5,8 tétrahydro-5,6,7,8 naphtyl-2) carbonyl-4 benzaldéhyde N,N-diméthylhydrazone de point de fusion: 122°C.

Le spectre $^1H$ R.M.N correspond à la structure attendue.

c) Préparation du (méthano-5,8 tétrahydro-5,6,7,8 naphtyl-2) carbonyl-4 benzaldéhyde.

Un mélange agité de 1,85g d'hydrazone obtenue ci-dessus en (b), de 2,8cm3 d'une solution aqueuse de glyoxal 6,2 M et de 5 gouttes d'acide chlorhydrique concentré dans 100cm3 de toluène est porté à une température de 60°C. La transformation de l'hydrazone est suivie en chromatographie couche mince. Lorsque le produit de départ est entièrement transformé, le milieu réactionnel est lavé à l'eau et la phase toluénique est séchée sur sulfate de magnésium. On obtient, après évaporation du toluène sous pression réduite, 1,26g de (méthano-5,8 tétrahydro-5,6,7,8 naphtyl-2) carbonyl-4 benzaldéhyde sous forme d'un liquide visqueux dont le spectre de $^1H$ R.M.N correspond à la structure attendue.

EXEMPLE IV

Préparation du (tétraméthyl-5,5,8,8 tétrahydro-5,6,7,8 naphtyl-2) carbonyl-4 benzaldéhyde.

Composé de formule IV dans laquelle A=-$(CH_2)_2$-, R′ et R″=oxo et $R'_7$=H

A un mélange de 14,7g (0.078 mole) de tétraméthyl-5,5,8,8 tétrahydro-5,6,7,8 naphtalène et de 16,3g (0,077 mole) de chlorure d'acide obtenu à l'exemple IIIa dans 500cm3 de dichloro-1,2 éthane anhydre, on ajoute sous agitation à une température inférieure à 5°C, 15,6g (0,117 mole) de chlorure d'aluminium par petites portions. l'agitation est poursuivie pendant 30mn à 5°C puis deux heures a température ordinaire, temps au bout duquel la réaction est terminée. Le mélange réactionnel de couleur rouge intense est alors versé sur de la glace et la phase organique est décantée, puis séchée sur sulfate de magnésium. Après évaporation du dichloro-1,2 éthane sous pression réduite, le produit obtenu est extrait à l'acétate d'éthyle. La phase organique est alors lavée à l'aide d'une solution aqueuse d'hydrogénocarbonate de sodium puis à l'eau et ensuite séchée sur sulfate de magnésium.

Après évaporation de l'acétate d'éthyle sous pression réduite, puis purification par chromatographie de gel de silice on obtient 9,25g de (tétraméthyl-5,5,8,8 tétrahydro-5,6,7,8 naphtyl-2) carbonyl-4 benzaldéhyde N,N-diméthylhydrazone sous forme de cristaux de point de fusion:119°C.

A une solution de 6,8g de l'hydrazone obtenue dans 250cm3 de toluène on ajoute 13,5cm3 d'une solution aqueuse de glyoxal 6,2 M et 2cm3 d'acide chlorhydrique concentré. Le mélange est alors porté pendant 4 heures à 60°C, puis à la température ordinaire et lavé avec 200cm3 d'eau. La phase toluénique est ensuite décantée, séchée sur sulfate de magnésium puis concentrée sous pression réduite.

On obtient 5,5g de (tétraméthyl-5,5,8,8 tétrahydro-5,6,7,8 naphtyl-2) carbonyl-4 benzaldéhyde sous forme d'une poudre jaune de point de fusion: 127-130°C.

Le spectre $^1$H R.M.N correspond à la structure attendue.

EXEMPLE V

Synthèse du (pentaméthyl-1,1,2,3,3 indanyl-5) carbonyl-4 benzoate de méthyle.

Composé de formule IV dans laquelle

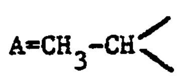 ,

R′ et R″=oxo et $R'_7$=-$OCH_3$.

A une suspension agitée à température ambiante de 5g (0,027 mole) de pentaméthyl-1,1,2,3,3 indane et de 5g (0,025 mole) du chlorure de l'acide méthoxy carbonyl-4 benzoïque dans 100cm3 de dichloro-1,2 éthane anhydre on ajoute par petites portions 6g de chlorure d'aluminium en poudre de manière à maintenir la température inférieure à 35°C.

L'agitation est maintenue pendant 1 heure jusqu'à disparition totale du produit de départ puis le milieu réactionnel est versé sur 150cm3 d'eau glacée et extrait au dichlorométhane.

La phase organique est lavée avec une solution aqueuse d'hydrogénocarbonate de sodium, puis à l'eau, séchée sur sulfate de magnésium et concentrée sous pression réduite.

Par purification par chromatographie sur gel de silice (éluant hexane/acétate d'éthyle 9:1) on obtient 6,5g de (pentaméthyl-1,1,2,3,3 indanyl-5) carbonyl-4 benzoate de méthyle de point de fusion: 146-147°C (après recristallisation dans l'hexane).

Analyse élémentaire: $C_{23}H_{26}O_3$

Calculé: C: 78,82 H: 7,48 O:13,70

Trouvé: 78,74 7,52 13,80

EXEMPLE VI

Synthèse de l'acide (pentaméthyl-1,1,2,3,3 indanyl-5) carbonyl-4 benzoïque.

Composé de formule IV dans laquelle

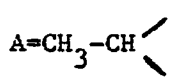

R′ et R″=oxo et $R'_7$=-OH.

Une suspension de 4,9g de (pentaméthyl-1,1,2,3,3 indanyl-5) carbonyl-4 benzoate de méthyle obtenu à l'exemple V dans 200cm3 d'alcool éthylique et 75cm3 d'une solution aqueuse de potasse 6N est portée à 40°C pendant environ 3 heures jusqu'à disparition totale du produit de départ. Après évaporation de l'alcool sous pression réduite, la phase aqueuse est diluée avec 500cm3 d'eau, refroidie à 0°C et acidifiée à l'acide chlorhydrique concentré. Le précipité obtenu est filtré, séché et recristallisé dans un mélange toluène-hexane. On récupère ainsi 3,9g d'une poudre blanche de point de fusion: 164-165°C.

Le spectre $^1H$ R.M.N 80 MHz est conforme à la structure de l'acide (pentaméthyl-1,1,2,3,3 indanyl-5) carbonyl-4 benzoïque.

Analyse élémentaire: $C_{22}H_{24}O_3$
Calculé: C: 78,54 H:7,19 O:14,27
Trouvé: 78,40 7,23 14,21

EXEMPLE VII

Synthèse du N-éthyl (pentaméthyl-1,1,2,3,3 indanyl-5) carbonyl-4 benzamide

Composé de formule IV dans laquelle

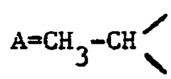

R' et R"=oxo et $R'_7$=$-NHC_2H_5$.

A une solution de 2,6g d'acide (pentaméthyl-1,1,2,3,3 indanyl-5) carbonyl-4 benzoïque obtenu à l'exemple VI dans 100cm3 de dichlorométhane anhydre on ajoute 2,5g de N,N'-carbonyldiimidazole. Après l'addition, on maintient l'agitation pendant 3 heures puis on refroidit le milieu réactionnel à 0°C et on ajoute 2,5cm3 d'éthylamine anhydre. On maintient l'agitation pendant 2 heures à température ambiante puis la solution est versée sur 200cm3 d'eau. La phase aqueuse est extraite au dichlorométhane et les phases organiques sont rassemblées, lavées, séchées sur sulfate de magnésium et concentrées sous pression réduite.

L'huile ainsi obtenue cristallise dans l'éther diisopropylique pour conduire à 1,9g du N-éthyl (pentaméthyl-1,1,2,3,3 indanyl-5) carbonyl-4 benzamide de point de fusion: 132-133°C.

Analyse élémentaire: $C_{24}H_{29}NO_2$
Calculé: C: 79,30 H, 8,04 N: 3,85 O:8,80
Trouvé: 79,08 8,06 3,92 8,74

EXEMPLE VIII

Synthèse du (pentaméthyl-1,1,2,3,3 indanyl-5)-1 (hydroxyméthyl-4 phényl)-1 méthanol.

Composé de formule II dans laquelle

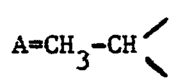

$R_1$=$R_2$=$R_3$=$R_4$=$CH_3$ R'=OH, R"=H et R=$-CH_2OH$.

A une suspension de 3,3g d'hydrure de lithium aluminium dans 200cm3 de tétrahydrofuranne anhydre maintenue à 0°C on ajoute goutte à goutte une solution de 10g de (pentaméthyl-1,1,2,3,3 indanyl-5) carbonyl-4 benzoate de méthyle obtenu à l'exemple V dans 100cm3 de tétrahydrofuranne. Après la fin de l'addition, on maintient l'agitation du milieu réactionnel, à température ambiante, pendant environ 3 heures jusqu'à disparition totale du produit de départ et des intermédiaires de réduction. Après addition de 50cm3 d'acétate d'éthyle pour détruire l'excès d'hydrure, la solution est versée sur 200cm3 d'eau, acidifiée avec de l'acide chlorhydrique 3N et extraite à l'acétate d'éthyle. Les phases organiques sont lavées, séchées sur sulfate de magnésium et concentrées sous pression réduite. On récupère 4,1g de (pentaméthyl-1,1,2,3,3 indanyl-5) (hydroxyméthyl-4 phényl)-1 méthanol qui cristallise dans l'hexane sous forme d'une poudre blanche de point de fusion: 107-108°C et dont le spectre $^1H$R.M.N 80 MHz est conforme à la structure attendue.

EXEMPLE IX

Synthèse du (pentaméthyl-1,1,2,3,3 indanyl-5) carbonyl-4 benzaldéhyde.

Composé de formule IV dans laquelle

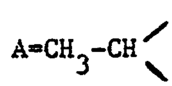

R' et R"=oxo et $R'_7$=H.

A une solution de 2g (0,0062 mole) de (pentaméthyl-1,1,2,3,3 indanyl-5) (hydroxyméthyl-4 phényl)-1 méthanol obtenu à l'exemple VIII dans 100cm3 de dichlorométhane anhydre on ajoute 3,4g de chlorochromate de pyridinium.

L'agitation est maintenue pendant environ 3 heures jusqu'à disparition totale du produit de départ puis, après addition de 200cm3 de dichlorométhane et de 20g environ de silice, la solution est filtrée, lavée avec une solution de chlorure d'ammonium et à l'eau puis séchée sur sulfate de magnésium et concentrée sous pression réduite. Après cristallisation dans un mélange hexane-éther diisopropylique, on obtient 1,2g d'une poudre blanche de point de fusion: 114-115°C dont le spectre $^{1}H$ R.M.N 80 MHz est conforme à la structure attendue.

Analyse élémentaire: $C_{22}H_{24}O_2$
Calculé: C: 82,46 H: 7,55 O: 9,99
Trouvé: 82,31 7,56 10,02

EXEMPLE X

Synthèse du (pentaméthyl-1,1,2,3,3 indanyl-5) hydroxyméthyl-4 benzoate de méthyle

Composé de formule IV dans laquelle

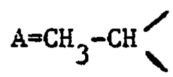

R"=H et R'7=-$OCH_3$.

A une suspension de 2g du (pentaméthyl-1,1,2,3,3 indanyl-5) carbonyl-4 benzoate de méthyle obtenu à l'exemple V dans 100cm3 de méthanol on ajoute par petites portions 0,7g de borohydrure de sodium en maintenant la température inférieure à 30°C. On maintient l'agitation à température ambiante pendant environ 3 heures jusqu'à disparition totale du produit de départ. Le mélange réactionnel est hydrolysé avec 100cm3 d'eau et acidifié avec une solution d'acide chlorhydrique 3N. Après évaporation sous pression réduite du méthanol, le produit attendu est extrait à l'acétate d'éthyle. Les phases organiques sont lavées, séchées et concentrées sous pression réduite. On récupère 1,7g d'une poudre blanche dont le spectre $^{1}H$ 80 MHz correspond à la structure du (pentaméthyl-1,1,2,3,3 indanyl-5) hydroxyméthyl-4 benzoate de méthyle. Par recristallisation d'un échantillon dans l'hexane on obtient des paillettes blanches de point de fusion: 126-127°C.

Analyse élémentaire: $C_{23}H_{28}O_3$
Calculé: C: 78,37 H: 8,00 O: 13,62
Trouvé: 78,33 7,93 13,71

EXEMPLE XI

Synthèse de l'acide (pentaméthyl-1,1,2,3,3 indanyl-5) hydroxyméthyl-4 benzoïque.

Composé de formule IV dans laquelle

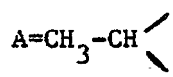

R'=OH, R"=H et R'7=-OH.

Une suspension de 1g de (pentaméthyl-1,1,2,3,3 indanyl-5) hydroxyméthyl-4 benzoate de méthyle obtenu à l'exemple X, dans un mélange de 100cm3 d'alcool éthylique et de 30cm3 d'une solution aqueuse de potasse 6N, est chauffée à 40°C pendant environ 1 heure jusqu'à disparition totale du produit de départ. L'alcool est évaporé sous pression réduite puis la phase aqueuse est diluée avec 300cm d'eau. Après avoir refroidi à 0°C et acidifié à l'aide d'acide chlorhydrique 3N, le précipité obtenu est filtré, séché et recristallisé dans un mélange toluène-hexane. On récupère 600mg d'une poudre blanche de point de fusion: 187-188°C.

Le spectre $^{1}H$ R.M.N 80 MHz est conforme à la structure de l'acide (pentaméthyl-1,1,2,3,3 indanyl-5) hydroxyméthyl-4 benzoïque.

Analyse élémentaire: $C_{22}H_{26}O_3$
Calculé: C: 78,07 H: 7,74 O: 14,18
Trouvé: 77,87 7,67 14,31

EXEMPLE XII

Acide (tétraméthyl-5,5,8,8 tétrahydro-5,6,7,8 naphtyl-2) hydroxyméthyl-4 benzoïque

Composé de formule IV dans laquelle A=-$(CH_2)_2$-, R'=OH, R"=H et $R'_7$=-OH.

A une solution agitée à température ambiante de 0,5g de l'acide (tétraméthyl-5,5,8,8 tétrahydro-5,6,7,8 naphtyl-2) carbonyl-4 benzoïque obtenu à l'exemple II, dans 50cm3 de méthanol, on ajoute goutte à goutte par petites portions 0,25g de borohydrure de sodium. L'agitation est maintenue pendant environ 1 heure jusqu'à disparition totale du produit de départ. Le milieu réactionnel est alors hydrolysé avec 100cm3 d'eau puis acidifié avec de l'acide chlorhydrique concentré.

Après évaporation sous pression réduite du méthanol la phase aqueuse est diluée avec 50cm3 d'eau et extraite à l'acétate d'éthyle. Les phases organiques sont lavées, séchées sur sulfate de magnésium et concentrées sous pression réduite. Par recristallisation dans un mélange toluène-hexane on récupère 200mg d'une poudre blanche de point de fusion:177-178°C, dont le spectre $^1$HR.M.N 80 MHz correspond à la structure de l'acide (tétraméthyl-5,5,8,8 tétrahydro-5,6,7,8 naphtyl-2) hydroxyméthyl-4 benzoïque.

Analyse élémentaire: $C_{22}H_{26}O_3$
Calculé: C: 78,07 H: 7,74 O: 14,18
Trouvé: 78,00 7,76 14,23

EXEMPLE XIII

Préparation du trans (méthano-5,8 tétrahydro-5,6,7,8 naphtyl-2) carbonyl-4 cinnamate d'éthyle

Composé de formule III dans laquelle: A=-$(CH_2)_2$-, $R_1$ et $R_3$=-$CH_2$-, $R_2$=$R_4$=$R_5$=$R_6$=H, R' et R"=oxo et R=-$CO_2C_2H_5$.

A une solution agitée à la température ordinaire de 1,83cm3 (9 mmoles) de triéthylphosphono acétate dans 75cm3 de T.H.F anhydre, on ajoute par petites fractions 0,45g d'hydrure de sodium. On ajoute ensuite, à l'abri de la lumière, une solution de 1,7g (6 mmoles) de (méthano-5,8 tétrahydro-5,6,7,8 naphtyl-2) carbonyl-4 benzaldéhyde, obtenu à l'exemple III, dans 10cm3 de T.H.F. L'évolution de la réaction est suivie par chromatographie sur couche mince. A la fin de la réaction, le mélange est versé sur de la glace puis extrait trois fois avec 100cm3 d'acétate d'éthyle. Les phases organiques sont rassemblées, lavées avec une solution saturée de chlorure d'ammonium puis à l'eau et enfin séchées sur sulfate de magnésium. L'acétate d'éthyle est rectifié sous pression réduite et le produit attendu cristallise par agitation dans l'hexane.

On obtient ainsi 1,5g de trans (méthano-5,8 tétrahydro-5,6,7,8 naphtyl-2) carbonyl-4 cinnamate d'éthyle sous forme de cristaux blancs de point de fusion: 92°C.

La spectre $^1$H R.M.N correspond à la structure attendue.

EXEMPLE XIV

Préparation de l'acide trans (méthano-5,8 tétrahydro-5,6,7,8 naphtyl-2) carbonyl-4 cinnamique.

Composé de formule III dans laquelle: A=-$(CH_2)_2$-, $R_1$ et $R_3$=-$CH_2$-, $R_2$=$R_4$=$R_5$=$R_6$=H, R' et R"=oxo et R=-$CO_2$H.

Une suspension agitée, à l'abri de la lumière, de 1,3g de trans (méthano-5,8 tétrahydro-5,6,7,8 naphtyl-2) carbonyl-4 cinnamate d'éthyle obtenu à l'exemple XIII dans 50cm3 d'éthanol et 50cm3 d'une solution aqueuse de potasse 6N est porté pendant environ une heure à une température de 50°C. Après évaporation de l'éthanol sous pression réduite, la phase aqueuse est acidifiée à température ordinaire et sous agitation, par addition d'acide chlorhydrique. Le précipité formé est alors essoré, séché puis, recristallisé dans un mélange toluène-hexane. On obtient 0,750g d'acide trans (méthano-5,8 tétrahydro-5,6,7,8 naphtyl-2) carbonyl-4 cinnamique sous forme de cristaux blancs de point de fusion: 189°C.

Le spectre de $^1$H R.M.N correspond à la structure attendue.

Analyse élémentaire: $C_{21}H_{18}O_3$
Calculé: C: 79,22 H: 5,70 O: 15,08
Trouvé: 79,20 5,75 15,11

EXEMPLE XV

Préparation du trans (tétraméthyl-5,5,8,8 tétrahydro-5,6,7,8 naphtyl-2) carbonyl-4 cinnamate d'éthyle.

Composé de formule V dans laquelle A=-$(CH_2)_2$-, $R_6$=H, R' et R"=oxo et $R'_7$=-$OC_2H_5$.

A une solution de 4,8cm3 (24,3 mmoles) de triéthylphosphonoacétate dans 150cm3 de T.H.F. anhydre, on ajoute par petites fractions 1,17g (24 mmoles) d'hydrure de sodium. A ce mélange, agité à la température ordinaire et à l'abri de la lumière, on introduit goutte à goutte une solution de 5,2g (16,2 mmoles) de (tétraméthyl-5,5,8,8 tetrahydro-5,6,7,8 naphtyl-2) carbonyl-4 benzaldéhyde obtenu à l'exemple IV dans

50cm3 de T.H.F anhydre. L'agitation est maintenue à l'abri de la lumière jusqu'à transformation totale du produit de départ. Le milieu réactionnel est ensuite versé sur une solution aqueuse de chlorure d'ammonium et le mélange est extrait à l'acétate d'éthyle. La phase organique est décantée, lavée à l'eau et séchée sur

Il est ensuite essoré puis recristallisé dans un mélange toluène-hexane.

On isole ainsi 3g de trans (tétraméthyl-5,5,8,8 tétrahydro-5,6,7,8 naphtyl-2) carbonyl-4 cinnamate d'éthyle sous forme de cristaux blancs de point de fusion: 97°C. Par passage du filtrat sur colonne de chromatographie de gel de silice et élution de produit attendu au mélange hexane-acétate d'éthyle 1g de produit pur supplémentaire est obtenu.

Analyse élémentaire: $C_{26}H_{30}O_3$
Calculé: C: 79,96 H: 7,74 O: 12,29
Trouvé: 80,01 7,77 12,20

EXEMPLE XVI

Préparation de l'acide trans (tétraméthyl-5,5,8,8 tétrahydro-5,6,7,8 naphtyl-2) carbonyl-4 cinnamique.

Composé de formule V dans laquelle: A=-$(CH_2)_2$-, $R_6$=H, R' et R"=oxo et $R'_7$=-OH.

Une suspension de 3g de trans (tétraméthyl-5,5,8,8 tétrahydro-5,6,7,8 naphtyl-2) carbonyl-4 cinnamate d'éthyle obtenu à l'exemple XV dans un mélange de 100cm3 d'éthanol et 100cm3 de potasse aqueuse 6N est agitée à 50°C, à l'abri de la lumière pendant deux heures. L'éthanol est ensuite éliminé par évaporation sous vide et la phase aqueuse résiduelle est acidifiée par addition d'acide chlorhydrique concentré. Après 30mn d'agitation, le précipité formé est essoré, séché, puis solubilisé dans le minimum de chlorure de méthylène et déposé sur une colonne de chromatographie de gel de silice. Le produit attendu est élué au mélange hexane-dichlorométhane (1-1).

Après évaporation de l'éluant on obtient l'acide trans (tétraméthyl-5,5,8,8 tétrahydro-5,6,7,8 naphtyl-2) carbonyl-4 cinnamique sous forme de cristaux blancs de point de fusion: 232°C.

Analyse élémentaire: $C_{24}H_{26}O_3$
Calculé: C: 79,53 H: 7,23 O: 13,24
Trouvé: 79,29 7,21 13,05

EXEMPLE XVII

Préparation du trans (tétraméthyl-5,5,8,8 tétrahydro-5,6,7,8 naphtyl-2) carbonyl-4 -méthyl cinnamate d'éthyle.

Composé de formule V dans laquelle: A=-$(CH_2)_2$-, $R_6$=-$CH_3$, R' et R"=oxo et $R'_7$=-$OC_2H_5$.

A une solution de 1,95g (8,2 mmoles) de triéthylphosphono-2 propionate dans 100cm3 de T.H.F anhydre, on ajoute sous agitation, par petites portions 0,44g (9 mmoles) d'hydrure de sodium. Après 30mn, on ajoute alors, à l'abri de la lumière, une solution de 1,75g (5,5 mmoles) de alors, à l'abri de la lumière, une solution de 1,75g (5,5 mmoles) de (tétraméthyl-5,5,8,8 tétrahydro-5,6,7,8 naphtyl-2) carbonyl-4 benzaldéhyde obtenu à l'exemple IV, dans 20cm3 de T.H.F.

Après 3 heures d'agitation le mélange réactionnel est versé sur de l'eau glacée et la solution obtenue est extraite trois fois à l'aide de 50cm3 d'acétate d'éthyle. Les phases organiques sont rassemblées, lavées avec 100cm3 d'eau, séchées sur sulfate de magnésium et concentrées sous pression réduite. Le produit attendu est purifié par passage sur colonne de gel de silice par élution au mélange hexane-acétate d'éthyle (95-5). Après évaporation de l'éluant sous pression réduite, le trans (tétraméthyl-5,5,8,8 tétrahydro-5,6,7,8 naphtyl-2) carbonyl-4 α-méthyl cinnamate d'éthyle est isolé sous forme de cristaux blancs de point de fusion:106°C.

Analyse élémentaire: $C_{27}H_{32}O_3$
Calculé: C: 80,16 H: 7,97 O: 11,87
Trouvé: 80,24 7,98 11,68

EXEMPLE XVIII

Préparation de l'acide dans (tétraméthyl-5,5,8,8 tétrahydro-5,6,7,8 naphtyl-2) carbonyl-4 α-méthyl cinnamique.

Composé de formule V dans laquelle: A=-$(CH_2)_2$-, $R_6$=-$CH_3$, R' et R"=oxo et $R'_7$=-OH.

Une suspension de 2,75 g de trans (tétraméthyl-5,5,8,8 tétrahydro-5,6,7,8 naphtyl-2) carbonyl-4 α-méthyl cinnamate d'éthyle obtenu à l'exemple XVII est agitée pendant 2 heures dans un mélange de 100cm3 d'éthanol et 25cm3 de potasse aqueuse 6N à environ 40°C. Après évaporation de l'éthanol sous pression réduite, le résidu est repris avec 200cm3 d'eau et acidifié avec de l'acide chlorhydrique concentré. L'acide attendu est filtré, lavé et séché. Par recristallisation dans un mélange toluène-hexane on récupère 1,9g d'acide dans (tétraméthyl-5,5,8,8 tétrahydro-5,6,7,8 naphtyl-2) carbonyl-4 α-méthyl cin-

namique de point de fusion: 205-206°C.
Analyse élémentaire: $C_{25}H_{28}O_3$
Calculé: C: 79,75 H: 7,50 O: 12,75
Trouvé: 79,25 7,52 12,39

EXEMPLE XIX

Préparation du (pentaméthyl-1,1,2,3,3 indanyl-5) carbonyl-4 α-méthyl cinnamate d'éthyle.

Composé de formule V dans laquelle:

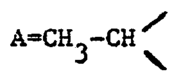

$R_6$=-$CH_3$, R' et R"=oxo et $R'_7$=-$OC_2H_5$.

A une solution de 1,5cm3 de triéthyl phosphono-2 propionate dans 100cm3 de T.H.F anhydre on ajoute par petites portions 0,4g d'hydrure de sodium. L'agitation est maintenue pendant environ 1 heure, puir à l'abri de la lumière, on ajoute quelques gouttes d'éther couronne et une solution de 1,4g de (pentaméthyl-1,1,2,3,3 indanyl-5) carbonyl-4 benzaldéhyde obtenu à l'exemple IX en solution dans 25cm3 de tétrahydrofuranne anhydre. A la fin de l'addition l'agitation est maintenue pendant 2 heures, puis le milieu réactionnel est versé sur une solution saturée de chlorure d'ammonium et extrait à l'acétate d'éthyle.

Les phases organiques sont lavées, séchées et concentrées sous pression réduite. Par cristallisation dans l'hexane on récupère 1,1g de (pentaméthyl-1,1,2,3,3 indanyl-5) carbonyl-4 α-méthyl cinnamate d'éthyle sous forme de poudre blanche de point de fusion: 89-90°C dont le spectre $^1H$ 80 MHz est conforme à la structure attendue.

Analyse élémentaire: $C_{27}H_{32}O_3$
Calculé: C: 80,16 H: 7,97 O: 11,87
trouvé: 79,70 8,08 11,75

EXEMPLE XX

Préparation de l'acide trans (pentaméthyl-1,1,2,3,3 indanyl-5) carbonyl-4 α-méthyl cinnamique.

Composé de formule V dans laquelle:

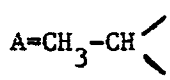

$R_6$=-$CH_3$ , R' et R"=oxo et $R'_7$=-OH.

Une suspension de 0,9g de (pentaméthyl-1,1,2,3,3 indanyl-5) carbonyl-4 α-méthyl cinnamate d'éthyle obtenu à l'exemple XIX est agitée pendant environ 1 heure dans un mélange de 100cm3 d'éthanol et 30cm3 de potasse aqueuse 6N à une température comprise entre 40 et 50°C. Après évaporation de l'éthanol sous pression réduite, la résidu est repris avec 500cm3 d'eau et acidifié avec de l'acide chlorhydrique 3N. L'acide attendu précipite. Il est filtré, lavé et séché.

Par recristallisation dans un mélange toluène-hexane on récupère 600mg d'une poudre blanche de point de fusion: 171-172°C,dont le spectre $^1H$ 80 MHz correspond à la structure de l'acide trans (pentaméthyl-1,1,2,3,3 indanyl-5) carbonyl-4 α-méthyl cinnamique.

Analyse élémentaire: $C_{25}H_{28}O_3$
Calculé: C: 79,75 H: 7,50 O: 12,75
Trouvé: 79,10 7,54 12,60

EXEMPLE XXI

Préparation du trans (tétraméthyl-5,5,8,8 tétrahydro-5,6,7,8 naphtyl-2) hydroxyméthyl-4 cinnamate d'éthyle.

Composé de formule V dans laquelle: A=-$(CH_2)_2$-, $R_6$=H ,R'=OH, R"=H et $R'_7$=-$OC_2H_5$.

1) Préparation du formyl-4 cinnamate d'éthyle

(a) mono N,N-diméthylhydrazino téréphtalaldéhyde.

A une solution de 75g de téréphtalaldéhyde dans 800cm3 de T.H.F anhydre on ajoute goutte à goutte

une solution de 42cm3 de N,N-diméthyl-hydrazine dans 50cm3 de T.H.F de manière à maintenir la température du milieu réactionnel inférieure à 30°C. A la fin de l'addition on maintient l'agitation pendant 2 heures jusqu'à disparition totale du téréphtalaldéhyde de départ. Après évaporation du T.H.F. et cristallisation du produit dans l'heptane on récupère 93g de mono N,N-diméthylhydrazinotérépthalaldéhyde contenant un peu de di N,N-diméthyl=hydrazinotéréphtalaldéhyde. Le produit obtenu est utilisé tel quel pour la suite de la réaction.

(b) (N,N-diméthylhydrazino) formyl-4 cinnamate d'éthyle.

A une solution de 23cm3 de triéthyl phosphonoacétate dans 400cm3 de T.H.F, on ajoute par petites portions 6g d'hydrure de sodium.

A la fin de l'addition on maintient l'agitation pendant 2 heures puis on ajoute, à l'abri de la lumière, 10g de mono N,N-diméthylhydrazino téréphtalaldehyde obtenu ci-dessus, en solution dans 100cm3 de T.H.F de manière à maintenir la température inférieure à 30°C. A la fin de l'addition, l'agitation est maintenue pendant environ 1 heure jusqu'à disparition totale de l'aldéhyde de départ. Le milieu réactionnel est versé sur une solution de chlorure d'ammonium et extrait à l'acétate d'éthyle. Les phases organiques sont lavées, séchées sur sulfate de magnésium et concentrées sous pression réduite. On récupère 10g d'une huile dont le spectre $^{1}H$ R.M.N 80 MHz correspond à la structure attendue et qui est utilisée sous forme brute pour la réaction suivante.

(c) Formyl-4 cinnamate d'éthyle

A une solution de 10g de N,N-dimethylhydrazino formyl-4 cinnamate d'éthyle obtenu ci-dessus dans 150cm3 de toluène on ajoute 28cm3 de glyoxal aqueux (6,2M) et environ 1cm3 d'acide chlorhydrique concentré. La solution est portée à 70°C pendant environ 2 heures jusqu'à disparition du produit de départ. La phase organique est décantée, lavée à l'eau, séchée sur sulfate de magnésium et concentrée sous pression réduite.

Après purification par chromatographie sur gel de silice (éluant: hexane-acétate d'éthyle 8-2) on récupère 4g de formyl-4 cinnamate d'éthyle sous forme d'une huile dont le spectre $^{1}H$ R.M.N 80 MHz correspond à la structure attendue.

2) Préparation du trans (tétraméthyl-5,5,8,8 tétrahydro-5,6,7,8 naphtyl-2) hydroxyméthyl-4 cinnamate d'éthyle.

On ajoute une solution de 5,2g (0,0195 mole) de tétraméthyl-5,5,8,8 tétrahydro-5,6,7,8 bromo-2 naphtalène dans 50cm3 de T.H.F anhydre à 550mg de magnésium, en maintenant le reflux jusqu'à disparition du magnésium. Le milieu réactionnel est alors refroidi à 0°C et on ajoute goutte à goutte une solution de 1,9g de formyl-4 cinnamate d'éthyle obtenu ci-dessus dans 20cm3 de T.H.F. A la fin de l'addition le mélange réactionnel est maintenu sous agitation pendant 1h30 à température ambiante. On verse le mélange réactionnel sur 200cm3 d'une solution de chlorure d'ammonium puis extrait à l'éther. On lave la phase organique, sèche sur sulfate de magnésium puis concentre sous pression réduite. Le produit attendu est purifié par chromatographie sur gel de silice (éluant: heptane-acétate d'éthyle 9-1) et on récupère 1,1g d'une huile dont le spectre $^{1}H$ R.M.N 80 MHz correspond à la structure du trans (tétraméthyl-5,5,8,8 tétrahydro-5,6,7,8 naphtyl-2) hydroxyméthyl-4 cinnamate d'éthyle.

EXEMPLE XXII

Préparation de l'acide trans (tétraméthyl-5,5,8,8 tétrahydro-5,6,7,8 naphtyl-2) hydroxyméthyl-4 cinnamique.

Composé de formule V dans laquelle: A=-$(CH_2)_2$-, $R_6$= H, R'=OH, R"=H et $R'_7$=-OH.

Une solution de 1,1g de trans (tétraméthyl-5,5,8,8 tétrahydro-5,6,7,8 naphtyl-2) hydroxyméthyl-4 cinnamate d'éthyle obtenu à l'exemple XXI est chauffée à 40°C dans un mélange de 100cm3 d'alcool éthylique et 75cm3 de potasse aqueuse 6N jusqu'à disparition du produit de départ. L'éthanol est évaporé sous pression réduite et le résidu est repris avec 300cm3 d'eau. Le mélange est refroidi à 0°C et acidifié par une solution d'acide chlorhydrique 3N. Le produit attendu est filtré, lavé et séché.

Par recristallisation dans un mélange toluène-hexane on récupère 800mg d'acide trans (tétraméthyl-5,5,8,8 tétrahydro-5,6,7,8 naphtyl-2) hydroxyméthyl-4 cinnamique de point de fuision: 199-200°C.

Analyse élémentaire: $C_{24}H_{28}O_3$

Calculé: C: 79,09 H: 7,74 O: 13,17

Trouvé: 79,09 7,67 12,98

EXEMPLE XXIII

Préparation du (hydroxy-2 éthyl)-4' pipérazino (tétraméthyl-5,5,8,8 tétrahydro-5,6,7,8 naphtyl-2) carbonyl-4 benzamide.

Composé de formule IV dans laquelle: A=-$(CH_2)_2$- R' et R"=oxo et

$R'_7$ = 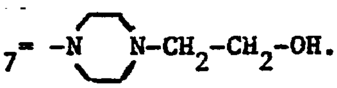

A une solution de 3g de l'acide (tétraméthyl-5,5,8,8 tétrahydro-5,6,7,8 naphtyl-2) carbonyl-4 benzoïque de l'exemple II dans 150cm3 de dichlorométhane anhydre on ajoute 2,9g de N,N'-carbonyldiimidazole. Après addition on maintient l'agitation pendant 3 heures puis on ajoute goutte à goutte 1,75g d'hydroxy-2 éthyl pipérazine et on maintient l'agitation encore pendant 2 heures jusqu'à disparition en C.C.M de l'acide de départ. Le mélange réactionnel est versé sur 200cm3 d'eau, extrait avec 3x100cm3 de dichlorométhane. Les phases organiques sont rassemblées, lavées, séchées sur sulfate de magnésium et concentrées sous pression réduite. Par purification sur gel de silice (éluant: acétate d'éthyle-méthanol 8-2) on récupère 2,2g d'(hydroxy-2 ethyl)-4' pipérazino (tétraméthyl-5,5,8,8 tétrahydro-5,6,7,8 naphtyl-2) carbonyl-4 benzamide qui cristallise dans un mélange toluène-hexane et dont le point de fusion est de: 118°C.

Analyse élémentaire: : $C_{28}H_{36}N_2O_3$
Calculé: C: 74,96 H: 8,09 N: 6,25 O: 10,70
Trouvé: 74,47 8,17 6,21 11,07

EXEMPLE XXIV

Préparation du N-(di-trifluorométhyl-3,5 phényl-1) (tétraméthyl-5,5,8,8 tétrahydro-5,6,7,8 naphtyl-2) carbonyl-4 benzamide.

Composé de formule IV dans laquelle: A=-$(CH_2)_2$- R' et R"=oxo et

$R'_7$ = 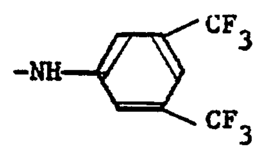

A une suspension de 1g de l'acide (tétraméthyl-5,5,8,8 tétrahydro-5,6,7,8 naphtyl-2) carbonyl-4 benzoïque de l'exemple II dans 75cm3 de diéthyloxyde anhydre on ajoute goutte à goutte 0,8cm3 de dicyclohexylamine en solution dans 10cm3 de diéthyloxyde. On maintient l'agitation pendant 2 heures puis on filtre le précipité obtenu. On récupère 1,5g d'une poudre blanche qui est mise en solution dans 75cm3 de dichloro-1,2 éthane anhydre. On additionne goutte à goutte une solution de 0,3cm3 de chlorure de thionyle dans 10cm3 de dichloro-1,2 éthane. A la fin de l'addition on maintient l'agitation pendant une nuit à température ambiante. Le mélange réactionnel est filtré et concentré sous pression réduite. On recupère environ 1g d'une huile jaune qui correspond au chlorure de l'acide (tétraméthyl-5,5,8,8 tétrahydro-5,6,7,8 naphtyl-2) carbonyl-4 benzoïque.

A une solution de 1g de chlorure d'acide obtenu précédemment dans 75$cm^3$ de dichloro-1,2 éthane on additionne goutte à goutte une solution de 0,7g de bis trifluorométhyl-3,5 aniline dans 10$cm^3$ de dichlorométhane. A la fin de l'addition le milieu réactionnel est maintenu sous agitation pendant 2 heures puis il est versé sur 200$cm^3$ d'eau et extrait au dichlorométhane. La phage organique est lavée avec une solution d'hydrogénocarbonate de sodium puis avec de l'eau, séchée sur sulfate de magnésium et concentrée sous pression réduite. Le résidu est repris dans l'hexane et filtré. On récupère 1,2g de N-(di-trifluorométhyl-3,5 phényl-1) (tétraméthyl-5,5,8,8 tétrahydro-5,6,7,8 naphtyl-2) carbonyl-4 benzamide dont le spectre $^1$HRMN 80MHZ est conforme à la structure attendue et dont le point de fusion est de: 215–216°C.

Analyse élémentaire: $C_{30}H_{27}F_6NO_2$
Calculé: C: 65,80 H: 4,97 F: 20,82 N: 2,56
Trouvé: C: 66,02 H: 4,93 F: 20,85 N: 2,48

EXEMPLE XXV

Préparation du N-éthyl trans (tétraméthyl-5,5,8,8 tétrahydro-5,6,7,8 naphtyl-2) carbonyl-4-méthyl cinnamide.

Composé de formule V dans laquelle: A= -$(CH_2)_2$-$R_6$= -$(CH_3)$R\` et R"=oxo et R\`$_7$= -$NHC_2H_5$.

A une solution agitée de 3g d'acide trans (tétraméthyl-5,5,8,8 tétrahydro-5,6,7,8 naphtyl-2) carbonyl-4-méthyl cinnamique (obtenu à l'exemple XVIII dans 100$cm^3$ de diméthylformamide anhydre agitée à la température ordinaire on ajoute 2,8g de N,N'-carbonyldiimidazole.

Le mélange est alors agité pendant trois heures a une température d'environ 50°C puis refroldi à 0°C température à laquelle on ajoute lentement 4,5$cm^3$ d'éthylamine anhydre. Le milieu réactionnel après une heure d'agitation est abandonné une nuit à température ambiante. Versé dans 200$cm^3$ d'eau puis extrait trois fois à l'aide de 100$cm^3$ d'acétate d'éthyle.

Les phases d'acétate d'éthyle sont rassemblées, lavées avec une solution de chlorure d'ammonium puis à l'eau et enfin séchées sur sulfate de magnésium. Après évaporation de l'acétate d'éthyle le produit brut obtenu est purifié par passage sur colonne de gel de silice et élué au mélange chlorure de méthylène-acétate d'éthyle (9-1).

Après distillation de l'éluant sous pression réduite et cristallisation dans l'hexane on obtient 1,9 g de N-éthyl trans/tétraméthyl-5,5,8,8 tétrahydro-5,6,7,8 naphtyl-2) carbonyl-4α-méthyl cinnamide sous forme d'une poudre jaune de point de fusion: 121°C.

Analyge élémentaire: $C_{27}H_{33}NO_2$.

Calculé C: 80,36 H: 8,24 N: 3,47 0: 7,93

Trouvé 80,27 8,33 3,40 8,08

heure d'agitation est abandonné une nuite à température ambiante. Versé dans 200 $cm^3$ d'eau puis extrait trois fois à l'aide de 100 $cm^3$ d'acétate d'éthyle.

EXEMPLE XXVI

Préparation du N-éthyl (tétraméthyl-5,5,8,8 tétrahydro-5,6,7,8 naphtyl-2) carbonyl-4 benzamide.

Composé de formule II dans laquelle A = $-(CH_2)_2$, $R_1=R_2=R_3=R_4=CH_3$ R' et R"= oxo et R=-CONHEt.

Dans une première étape on prépare le chlorure de l'acide N-éthyl carbamoyl-4 benzoïque en portant à ébullition une solution de 2g de cet acide dans 50cm3 de chlorure de thionyle pendant 4 heures.

Le chlorure de thionyle est alors éliminé par évaporation sous vide.

Les 2,2g de chlorure d'acide brut sont directement transformés en ajoutant à ce dernier 1,76g de tétraméthyl-5,5,8,8 tétrahydro-5,6,7,8 naphtalène dilué dans 40cm3 de dichloro-1,2 éthane anhydre.

Au mélange agité à 0°C, sous atmosphère inerte, on ajoute alors 1,9g de trichlorure d'aluminium. L'agitation est maintenue à cette température pendant 3 heures puis 5 heures à température ambiante.

Le mélange réactionnel est alors versé dans 200cm3 d'eau glacée puis extrait trois fois par 100cm3 de dichloro éthane. Les phases organiques sont rassemblées, lavées avec une solution aqueuse de chlorure d'aluminium puis à l'eau et enfin séchées sur sulfate de magnésium. Après évaporation du solvant on obtient 3,6g de produit brut. Le produit attendu est purifié par chromatographie sur gel de silice. Il est élué au mélange toluène/chlorure de méthylène/acétate d'éthyle (4-4-2) puis recristallisé dans un mélange hexane-éther ispropylique. On obtient 0,500g de N-éthyl (tétraméthyl-5,5,8,8 tétrahydro-5,6,7,8 naphtyl-2) carbonyl-4 benzamide sous forme de cristaux blancs de point de fusion : 102°C.

Analyse élémentaire: $C_{24}H_{29}NO_2$

Calculé: C: 79,30 H: 8,04 N: 3,85 O: 8,81

Trouvé: 79,74 7,94 3,67 8,77

EXEMPLES DE COMPOSITIONS

A. VOIE ORALE

Exemple 1 - Comprimé de 0,2g

| | |
|---|---|
| - Acide (tétraméthyl-5,5,8,8 tétrahydro-5,6,7,8 naphtyl-2) carbonyl-4 benzoïque............ | 0,010g |
| - Amidon.................................. | 0,115g |
| - Phosphate bicalcique...................... | 0,020g |
| - Silice.................................. | 0,020g |
| - Lactose................................. | 0,030g |
| - Talc.................................... | 0,010g |
| - Stéarate de magnésium..................... | 0,005g |

Dans cet exemple le composé actif peut être remplacé par la même quantité d'un des composés suivants:

- Acide trans (tétraméthyl-5,5,8,8 tétrahydro-5,6,7,8 naphtyl-2) carbonyl-4 cinnamique
- Acide trans (tétraméthyl-5,5,8,8 tétrahydro-5,6,7,8 naphtyl-2) carbonyl-4 α-méthyl cinnamique.

Exemple 2 - Suspension buvable en ampoules 5ml

| | |
|---|---|
| - Acide (pentaméthyl-1,1,2,3,3 indanyl-5) carbonyl-4 benzoïque | 0,010g |
| - Glycérine | 0,500g |
| - Sorbitol à 70% | 0,500g |
| - Saccharinate de sodium | 0,010g |
| - Parahydroxybenzoate de méthyle | 0,040g |
| - Arôme | q.s |
| - Eau purifiée qsp | 5,000g |

Dans cet exemple le composé actif peut être remplacé par la même quantité de l'acide trans (pentaméthyl-1,1,2,3,3 indanyl-5) carbonyl-4 α-méthyl cinnamique.

B. VOIE TOPIQUE

Exemple 3 - Onguent

| | |
|---|---|
| - (pentaméthyl-1,1,2,3,3 indanyl-5) carbonyl-4 α-méthyl cinnamate d'éthyle | 0,010g |
| - Huile de vaseline fluide | 9,100g |
| - Silice vendue par la Société DEGUSSA sous la dénomination de '"Aérosil 200" | 9,100g |
| - Myristate d'isopropyle qsp | 100,000g |

Dans cet exemple le composé actif peut être remplacé par la même quantite d'acide (tétraméthyl-5,5,8,8 tétrahydro-5,6,7,8 naphtyl-2) carbonyl-4 benzoïque.

Exemple 4 - Crème huile-dans-l'eau anionique

| | |
|---|---|
| - N-éthyl (pentaméthyl-1,1,2,3,3 indanyl-5) carbonyl-4 benzamide | 0,100g |
| - Dodécyl sulfate de sodium | 0,800g |
| - Glycérol | 2,000g |
| - Alcool stéarylique | 20,000g |
| - Triglycérides d'acides caprique/caprylique vendus par la Société DYNAMIT NOBEL sous la dénomination de "Miglyol 812" | 20,000g |
| - Conservateurs | q.s |
| - Eau déminéralisée qsp | 100,000g |

Dans cet exemple le composé actif peut être remplacé par la même quantité du composé suivant:
- Acide trans (tétraméthyl-5,5,8,8 tétrahydro-5,6,7,8 naphtyl-2) carbonyl-4 cinnamique.

Exemple 5 - Gel

| | |
|---|---|
| - N-éthyl (pentaméthyl-1,1,2,3,3 indanyl-5) carbonyl-4 benzamide...................... | 0,500g |
| - Hydroxypropyl cellulose vendue par la Société HERCULES sous le nom de "Klucel HF".............................. | 2,000g |
| - Eau/éthanol (50/50) qsp................... | 100,000g |

Dans cet exemple le composé actif peut être remplacé par 0,05g d'acide dans (tétraméthyl-5,5,8,8 tétrahydro-5,6,7,8 naphtyl-2) hydroxyméthyl-4 cinnamique ou encore par 0,1 g de N-éthyl trans (tétraméthyl-5,5,8,8 tétrahydro-5,6,7,8 napthyl-2) carbonyl-4 méthylcinnamide..

Exemple 6 - Crème anti-séborrhéique

| | |
|---|---|
| - Stéarate de polyoxyéthylène (40 moles d'oxyde d'éthylène) vendu sous la dénomination de "Myrj 52" par la Société "ATLAS"....... | 4,000g |
| - Mélange d'esters laurique de sorbitol et de sorbitan, polyoxyéthyléné à 20 moles d'oxyde d'éthylène vendu sous la denomination de "Tween 20" par la Société "ATLAS".......... | 1,800g |
| - Mélange de mono- et distéarate de glycérol vendu sous la dénomination de "GELEOL" par la Société "GATTEFOSSE"...................... | 4,200g |
| - Propylèneglycol.......................... | 10,000g |
| - Butylhydroxyanisole....................... | 0,010g |
| - Butylhydroxytoluène....................... | 0,020g |
| - Alcool céto-stéarylique................... | 6,200g |
| - Conservateurs............................. | q.s |
| - Perhydrosqualène.......................... | 18,000g |
| - Mélange de triglycérides caprylique/caprique vendu sous la dénomination de "Miglyol 812" par la Société "DYNAMIT NOBEL" ........... | 4,000g |
| - S-carboxyméthyl cystéine.................. | 3,000g |
| - Triéthanolamine 99%....................... | 2,500g |
| - Acide (pentaméthyl-1,1,2,3,3 indanyl-5) hydroxyméthyl-4 benzoïque................. | 0,100g |
| - Eau qsp.................................. | 100,000g |

Exemple 7 - Crème anti-séborrhéique

| | |
|---|---|
| - Stéarate de polyoxyéthylène (40 moles d'oxyde d'éthylène) vendu sous la dénomination de "Myrj 52" par la Société "ATLAS"........ | 4,000g |
| - Mélange d'esters laurique de sorbitol et de sorbitan, polyoxyéthylené à 20 moles d'oxyde d'éthylène, vendu sous le nom de "Tween 20" par la Société "ATLAS"........................... | 1,800g |
| - Mélange de mono- et distéarate de glycérol vendu sous la dénomination de "GELEOL" par la Société "GATTEFOSSE"....... | 4,200g |
| - Propylèneglycol........................... | 10,000g |
| - Butylhydroxyanisole....................... | 0,010g |
| - Butylhydroxytoluène....................... | 0,020g |
| - Alcool cétostéarylique.................... | 6,200g |
| - Conservateurs............................. | qsp |
| - Perhydrosqualène.......................... | 18,000g |
| - Mélange de triglycérides caprylique/caprique vendu sous la dénomination de "Miglyol 812" par la Société "DYNAMIT NOBEL"............ | 4,000g |
| - Amino-5 carboxy-5 thia-3 pentanoate de benzylthio-2 éthylammonium................ | 3,000g |
| - Acide (tétraméthyl-5,5,8,8 tétrahydro-5,6,7,8 naphtyl-2) hydroxyméthyl -4 benzoïque..... | 0,500g |
| - Eau qsp................................... | 100,000g |

Exemple 8 - Lotion pour les cheveux

| | |
|---|---|
| - Propylèneglycol.............................. | 20,000g |
| - Ethanol....................................... | 34,870g |
| - Polyéthylèneglycol de masse moléculaire 400. | 40,000g |
| - Eau............................................ | 4,000g |
| - Butylhydroxyanisole........................ | 0,010g |
| - Butylhydroxytoluène........................ | 0,020g |
| - Acide (tétraméthyl-5,5,8,8 tétrahydro-5,6,7,8 naphtyl-2) (hydroxyméthyl)-4 benzoïque..... | 0,100g |
| - Minoxidil..................................... | 1,000g |

Dans cet exemple le composé actif peut être remplacé par 0,05g de l'un des composés suivants:
-N-éthyl trans (tétraméthyl-5,5,8,8 tétrahydro-5,6,7,8 naphtyl-2) carbonyl-4 α-méthyl cinnamide
- Acide trans (tétraméthyl-5,5,8,8 tétrahydro-5,6,7,8 naphtyl-2) hydroxyméthyl-4 cinnamique.

Exemple 9 - Gel anti-acné

| | |
|---|---|
| - Acide (pentaméthyl-1,1,2,3,3 indanyl-5) carbonyl-4 benzoïque........................ | 0,100g |
| - Alcool isopropylique........................ | 40,000g |
| - Polymère de l'acide acrylique vendu sous la dénomination "CARBOPOL 940" par la Société "GOODRICH CHEMICAL COMPANY"................ | 1,000g |
| - Triéthanolamine 99%........................ | 0,600g |
| - Butylhydroxyanisole........................ | 0,010g |
| - Butylhydroxytoluène........................ | 0,020g |
| - Tioxolone.................................... | 0,500g |
| - Propylèneglycol.............................. | 8,000g |
| - Eau purifiée qsp............................ | 100,000g |

Dans cet exemple le composé actif peut être remplacé par la même quantité de N-éthyl trans (tétraméthyl-5,5,8,8 tétrahydro-5,6,7,8 naphtyl-2) carbonyl-4 méthyl cinnamide ou par 0,01 g d'acide trans (tétraméthyl-5,5,8,8 tétrahydro-5,6,7,8 naphtyl-2) carbonyl-4 cinnamique.

**Revendications pour les etats contractants: BE, CH, DE, FR, GB, IT, LI, NL, SE**

1. Composés bicycliques aromatiques, caractérisés par le fait qu'ils répondent à la formule suivante:

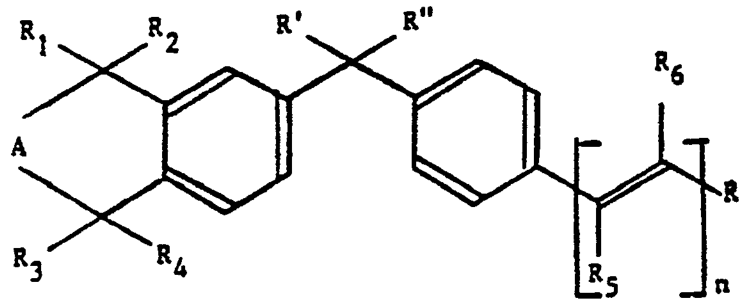

(I)

dans laquelle:
n est 0 ou 1,
R′ représente un atome d'hydrogène, un radical OH, un radical alkoxy ayant de 1 à 4 atomes de carbone, ou un radical acyloxy ayant de 1 à 4 atomes de carbone,
R″ représente un atome d'hydrogène ou un radical alkoxy ayant de 1 à 4 atomes de carbone,
ou R′ et R″, pris ensemble forment un radical oxo (=O), méthano (=$CH_2$) ou hydroxyimino (=N-OH),
R représente le radical -$CH_2OH$ ou le radical -$COR_7$,
$R_7$ représentant un atome d'hydrogène, le radical -$OR_8$ ou le radical

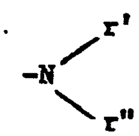

$R_8$ représentant un atome d'hydrogène, un radical alkyle ayant de 1 à 20 atomes de carbone, monohydroxyalkyle, polyhydroxyalkyle, aryle ou aralkyle éventuellement substitué(s) ou un reste d'un sucre ou encore le radical

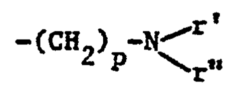

p étant 1, 2 ou 3 et r′ et r″ représentant un atome d'hydrogène, un radical alkyle inférieur, un radical monohydroxyalkyle éventuellement interrompu par un hétéroatome, un radical polyhydroxyalkyle, un radical aryle ou benzyle éventuellement substitué(s), un reste d'aminoacide ou de sucre aminé ou pris ensemble forment avec l'atome d'azote auquel ils sont rattachés, un hétérocycle,
A représente un radical méthylène ou diméthylène, substitué ou non par un radical alkyle inférieur,
$R_1$, $R_2$, $R_3$ et $R_4$ représentent un atome d'hydrogène ou un radical alkyle inférieur,
$R_1$ et $R_3$ pris ensemble pouvant former un radical méthylène ou diméthylène, lorsque A représente un radical diméthylène,
$R_5$ et $R_6$ représentent un atome d'hydrogène ou un radical méthyle, et les sels desdits composés ainsi que leurs isomères optiques et géometriques.

2. Composés selon la revendication 1, caractérisés par le fait que les radicaux alkyles inférieurs et ceux ayant jusqu'à 20 atomes de carbone sont pris dans le groupe constitué par les radicaux méthyle, éthyle, isopropyle, butyle, tertiobutyle, éthyl-2 hexyle, isooctyle, dodécyle, hexadécyle et octadécyle.

3. Composés selon la revendication 1, caractérisés par le fait que le radical monohydroxyalkyle est le radical hydroxy-2 éthyle, hydroxy-2 propyle ou hydroxy-2 éthoxyéthyle.

4. Composés selon la revendication 1, caractérisés par le fait que le radical polyhydroxyalkyle est le radical dihydroxy-2,3 propyle, dihydroxy-1,3 propyl-2 ou le reste du pentaérythritol.

5. Composés selon la revendication 1, caractérisés par le fait que le radical alkoxy est le radical méthoxy, isopropoxy, butoxy ou tert-butoxy.

6. Composés selon la revendication 1, caractérisés par le fait que le radical aryle est un radical phényle éventuellement substitué par un atome d'halogène, -OH, -$NO_2$, un radical alkyle inférieur, un radical trifluorométhyle ou une fonction acide carboxylique.

7. Composés selon la revendication 1, caractérisés par le fait que le radical aralkyle est le radical benzyle ou phénéthyle.

8. Composés selon la revendication 1, caractérisés par le fait que le reste d'un sucre dérive du glucose, du mannose, de l'érythrose ou du galactose.

9. Composés selon la revendication 1, caractérisés par le fait que le reste de sucres aminés dérive de glucosamine, de galactosamine, de mannosamine ou de méglumine.

10. Composés selon la revendication 1, caractérisés par le fait que les radicaux r' et r'' pris ensemble forment, avec l'atome d'azote auquel il sont rattachés, un radical pipéridino, pipérazino, morpholino, pyrrolidino ou (hydroxy-2 éthyl)-4 pipérazino.

11. Composés selon l'une quelconque des revendications 1 à 9, caractérisés par le fait qu'ils répondent à la formule suivante:

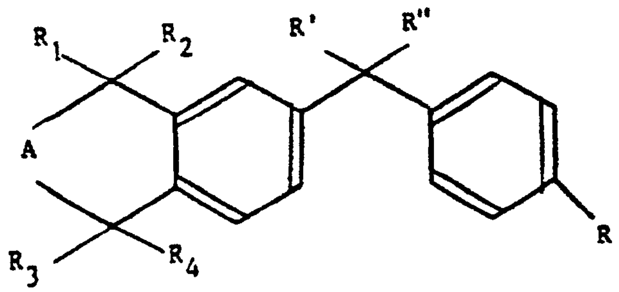

(II)

dans laquelle:
A, R, $R_1$ a $R_4$ , R' et R'' ont les mêmes significations que celles données à la revendication 1.

12. Composés selon l'une quelconque des revendications 1 à 9, caractérisés par le fait qu'ils répondent à la formule suivante:

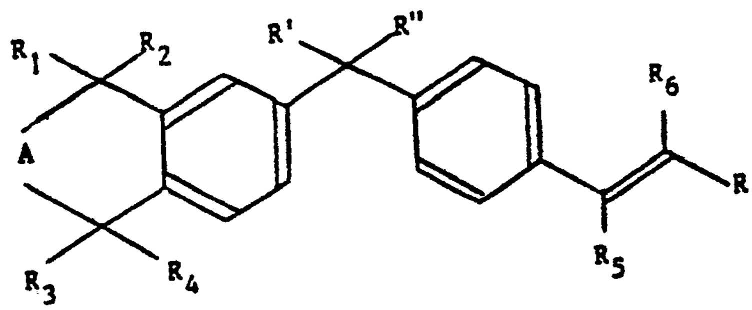

(III)

dans laquelle:
A, R, $R_1$ à $R_6$, R' et R'' ont les mêmes significations que celles données à la revendication 1.

13. Composés selon l'une quelconque des revendications 1 à 10, caractérisés par le fait qu'ils répondent à la formule suivante:

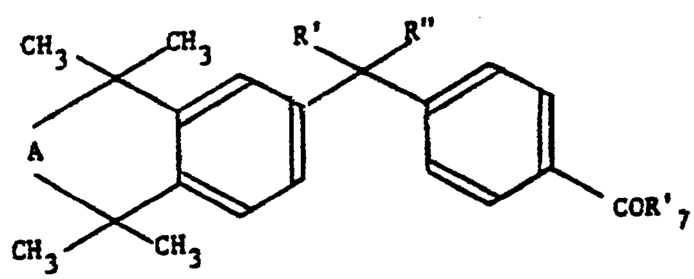

(IV)

dans laquelle:
A représente le radical

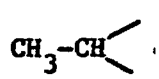

ou le radical diméthylène,

R′ représente un radical OH et R″ représente un atome d'hydrogène ou R′ et R″, pris ensemble, forment un radical oxo (=O),
et $R'_7$ représente un atome d'hydrogène, le radical $-OR'_8$ ou le radical

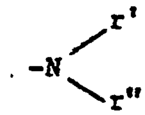

$R'_8$ représentant un atome d'hydrogène ou un radical alkyle inférieur,
r′ représentant un atome d'hydrogène et r″ représentant un radical alkyle inférieur ou un radical phényle substitué ou r′ et r″ pris ensemble forment avec l'atome d'azote auquel ils sont rattachés un radical (hydroxy-2 éthyl)-4 pipérazino.

14. Composés selon l'une quelconque des revendications 1 à 9 ou selon la revendication 11, caractérisés par le fait qu'ils répondent à la formule suivante:

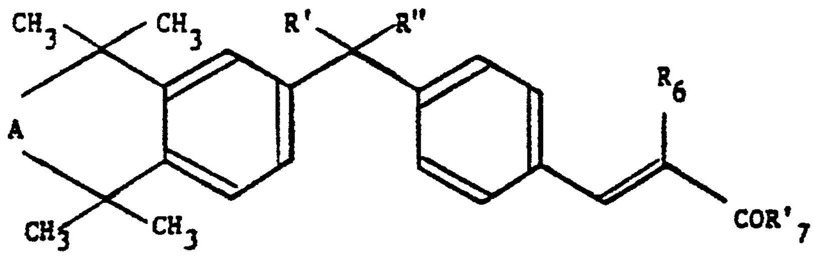

(V)

dans laquelle:
A représente le radical

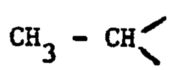

ou le radical diméthylène,
R′ représente un radical OH et R″ un atome d'hydrogène ou R′ et R″, pris ensemble, forment un radical oxo (=O),
$R_6$ représente un atome hydrogène ou un radical méthyle, et $R'_7$ représente $-OR'_8$ ou le radical

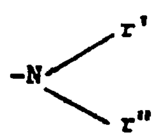

$R'_8$ représentant un atome d'hydrogène ou un radical alkyle inférieur.
r′ représentant un atome d'hydrogène et r″ représentant un radical alkyle inférieur,

15. Composés selon l'une quelconque des revendications 1 à 13, caractérisés par le fait qu'ils sont pris dans le groupe constitué par:
(1) le (tétraméthyl-5,5,8,8 tétrahydro-5,6,7,8 naphtyl-2) carbonyl-4 benzoate de méthyle,
(2) l'acide (tétraméthyl-5,5,8,8 tétrahydro-5,6,7,8 naphtyl-2) carbonyl-4 benzoïque,
(3) le (méthano-5,8 tétrahydro-5,6,7,8 naphtyl-2) carbonyl-4 benzaldéhyde,
(4) le (tétraméthyl-5,5,8,8 tétrahydro-5,6,7,8 naphtyl-2) carbonyl-4 benzaldéhyde,
(5) le (pentaméthyl-1,1,2,3,3 indanyl-5) carbonyl-4 benzoate de méthyle,
(6) l'acide (pentaméthyl-1,1,2,3,3 indanyl-5) carbonyl-4 benzoïque,
(7) le (pentaméthyl-1,1,2,3,3 indanyl-5) carbonyl-4 benzaldéhyde,
(8) le (pentaméthyl-1,1,2,3,3 indanyl-5)-1 (hydroxyméthyl-4 phényl)-1 méthanol,
(9) l'acide (tétraméthyl-5,5,8,8 tétrahydro-5,6,7,8 naphtyl-2) hydroxyméthyl-4 benzoïque,
(10) l'acide (pentaméthyl-1,1,2,3,3 indanyl-5) hydroxyméthyl-4 benzoïque,
(11)le (pentaméthyl-1,1,2,3,3 indanyl-5) hydroxyméthyl-4 benzoate de méthyle,
(12) le N-éthyl (tétraméthyl-5,5,8,8 tétrahydro-5,6,7,8 naphtyl-2) carbonyl-4 benzamide,
(13) le N-éthyl (pentaméthyl-1,1,2,3,3 indanyl-5) carbonyl-4 benzamide.
(14) le trans (méthano-5,8 tétrahydro-5,6,7,8 naphtyl-2) carbonyl-4 cinnamate d'éthyle,
(15) l'acide trans (méthano-5,8 tétrahydro-5,6,7,8 naphtyl-2) carbonyl-4 cinnamique,
(16) le trans (tétraméthyl-5,5,8,8 tétrahydro-5,6,7,8 naphtyl-2) carbonyl-4 cinnamate d'éthyle,

(17) l'acide trans (tétraméthyl-5,5,8,8 tétrahydro-5,6,7,8 naphtyl-2) carbonyl-4 cinnamique,
(18) le trans (tétraméthyl-5,5,8,8 tétrahydro-5,6,7,8 naphtyl-2) carbonyl-4 α-méthyl cinnamate d'éthyle,
(19) l'acide trans (tétraméthyl-5,5,8,8 tétrahydro-5,6,7,8 naphtyl-2) carbonyl-4 α-méthyl cinnamique,
(20) le trans (pentaméthyl-1,1,2,3,3 indanyl-5) carbonyl-4 α-méthyl cinnamate d'éthyle,
(21) l'acide trans (pentaméthyl-1,1,2,3,3 indanyl-5) carbonyl-4 α-methyl cinnamique,
(22) le trans (tétraméthyl-5,5,8,8 tétrahydro-5,6,7,8 naphtyl-2) hydroxyméthyl-4 α-méthyl cinnamate d'éthyle,
(23) l'acide trans (tétraméthyl-5,5,8,8 tétrahydro-5,6,7,8 naphtyl-2) hydroxyméthyl-4 α-méthyl cinnamique,
(24) le N-éthyl trans (tétraméthyl-5,5,8,8 tétrahydro-5,6,7,8 naphtyl-2) hydroxyméthyl-4 α-méthyl cinnamide,
(25) le N-éthyl trans (tétraméthyl-5,5,8,8 tétrahydro-5,6,7,8 naphtyl-2) carbonyl-4 α-méthyl cinnamide,
(26) le N-éthyl trans (pentaméthyl-1,1,2,3,3 indanyl-5) carbonyl-4 α-méthyl cinnamide,
(27) le trans (tétraméthyl-5,5,8,8 tétrahydro-5,6,7,8 naphtyl-2) hydroxyméthyl-4 cinnamate d'éthyle,
(28) l'acide trans (tétraméthyl-5,5,8,8 tétrahydro-5,6,7,8 naphtyl-2) hydroxyméthyl-4 cinnamique,
(29) l'(hydroxy-2 éthyl)-4' pipérazino (tétraméthyl-5,5,8,8 tétrahydro-5,6,7,8 naphtyl-2) carbonyl-4 benzamide,
(30) le N-(ditrifluorométhyl-3,5 phényl-1) (tétraméthyl-5,5,8,8 tétrahydro-5,6,7,8 naphtyl-2) carbonyl-4 benzamide.

16. Procédé de préparation des composés selon l'une quelconque des revendications 1 à 15, caractérisé par le fait qu'il consiste à faire réagir en milieu solvant organique un halogénure tel qu'un chlorure d'acide correspondant à la formule suivante:

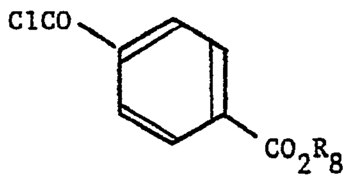

(1)

sur un composé aromatique correspondant à l'une des formule suivantes:

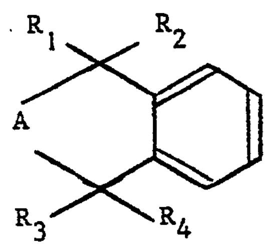

(2)

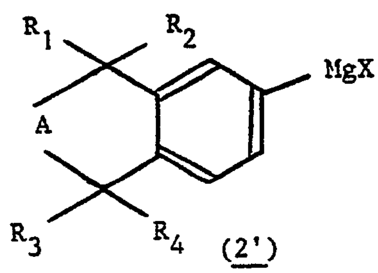

(2')

dans lesquels:
A, $R_1$ à $R_4$ ont les mêmes significations que celles données à la revendication 1, $R_8$ étant un radical alkyle ayant de 1 à 20 atomes de carbone, et X est Br ou Cl, que l'on procède, si nécessaire à la saponification du céto-ester obtenu en céto-acide correspondant et à la transformation subséquente dudit céto-acide en amide correspondant par action d'une amine de formule:

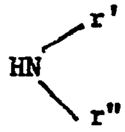

dans laquelle:
r' et r" ont les mêmes significations que celles données à la revendication 1, ou à la transformation subséquente dudit céto-acide en hydroxyacide ou en diol et à l'oxydation éventuelle du diol en céto-aldéhyde correspondant.

17. Procédé selon la revendication 16, caractérisé par le fait que la réaction de condensation, avec le composé aromatique (2), est effectuée en présence de chlorure d'aluminium anhydre dans du dichloro-1,2 éthane à une température comprise entre 0 et 25°C sous agitation.

18. Procédé selon la revendication 16, caractérisé par le fait que la condensation du chlorure d'acide sur l'organo magnésien (2') est effectuée dans le THF à une température d'environ 0°C.

19. Procédé selon la revendication 16, caractérisé par le fait que la préparation de l'amide est effectuée en présence de N,N'-carbonyl diimidazole.

20. Procédé selon la revendication 16, caractérisé par le fait que la réduction du céto-acide en hydroxy-acide correspondant est effectuée en présence de borohydrure de sodium dans le T.H.F.

21. Procédé selon la revendication 16, caractérisé par le fait que le céto-aldéhyde est obtenu par oxydation du diol à l'aide de chlorochromate de pyridinium, ledit diol correspondant résultant d'une réaction de réduction du céto-acide en présence d'hydrure de lithium aluminium.

22. Procédé de préparation des composés de formule (I) dans laquelle n = O et R=CHO, caractérisé par le fait qu'il consiste à faire réagir en milieu solvant organique dans les conditions de la réaction de Friedel-Crafts un chlorure d'acide de formule suivante:

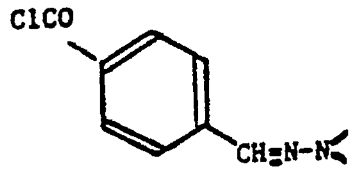

sur un composé aromatique de formule:

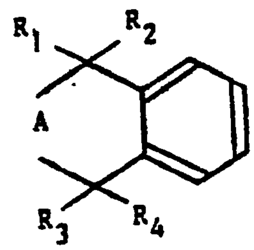

et que l'on procède ensuite à la libération de la fonction aldéhyde par échange avec le glyoxal pour conduire au céto-aldéhyde attendu.

23. Procédé de préparation des composés de formule:

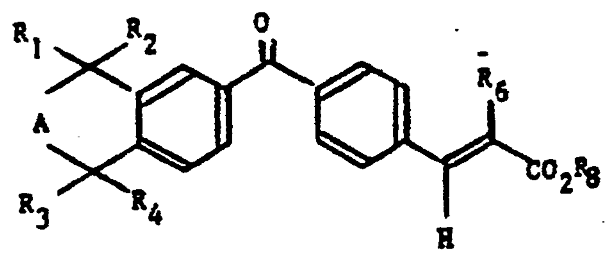

dans laquelle:
$R_1$ à $R_4$, $R_6$ et $R_8$ ayant les mêmes significations qu'à la revendication 1,
caractérisé par le fait que l'on fait réagir un céto-aldéhyde tel qu'obtenu aux revendications 16 ou 22 et ayant la formule suivante:

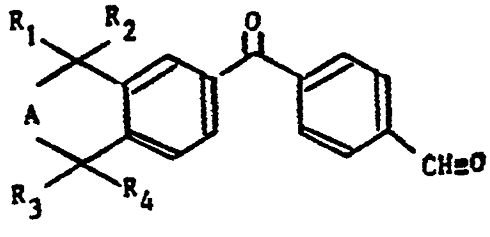

dans laquelle:

A et $R_1$ à $R_4$ ont les mêmes significations qu'à la revendication 1, avec un phosphonoacétate d'alkyle de formule:

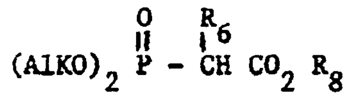

en présence d'hydrure de sodium dans le T.H.F et que l'on soumet le céto-ester insaturé obtenu aux conditions conventionnelles de réaction permettant d'accéder aux différentes significations des radicaux de la formule (I) selon la revendication 1.

24. Médicament, caractérisé par le fait qu'il est un composé de formule (I), selon l'une quelconque des revendications 1 à 15 ou obtenu selon l'une quelconque des revendications 16 à 23.

25. Médicament selon la revendication 24, caractérisé par le fait qu'il est administré à une dose journalière d'environ 2µg/kg à 2mg/kg de poids corporel.

26. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 15, ou obtenu selon l'une quelconque des revendications 16 à 23.

27. Composition selon la revendication 26, caractérisée par le fait qu'elle se présente sous une forme appropriée pour une application topique ou oculaire et contient de 0,0005 à environ 5% en poids d'un composé de formule (I).

28. Utilisation d'un composé selon l'une quelconque des revendications 1 à 15 ou obtenu selon l'une quelconque des revendications 16 à 23 pour la préparation d'une composition pharmaceutique destinée au traitement des affections dermatologiques, respiratoires ainsi qu'ophtalmologiques.

29. Composition cosmétique pour l'hygiène corporelle et capillaire, caractérisée par le fait qu'elle contient dans un véhicule cosmétique approprié, au moins un composé de formule (I) selon l'une quelconque des revendications 1 à 15 ou obtenu selon l'une quelconque des revendications 16 à 23.

30. Composition cosmétique selon la revendication 29, caractérisée par le fait qu'elle contient le composé de formule (I) à une concentration comprise entre 0,0005 et 2% et de préférence entre 0,01 et 1% en poids.

31. Composition selon l'une quelconque des revendications 26 ou 29, caractérisée par le fait qu'elle contient en outre au moins un additif inerte ou un additif pharmacodynamiquement ou cosmétiquement actif tel qu'un agent hydratant, un agent antiséborrhéique, un agent antiacnéique, un antibiotique, un agent favorisant la repousse des cheveux, un agent anti-inflammatoire, un caroténoïde, un agent antipsoriasique, un agent de sapidité, un agent conservateur, un agent stabilisant, un agent régulateur d'humidité, un agent régulateur de pH, un agent modificateur de pression osmotique, un agent émulsionnant, un filtre UV-A ou UV-B ou un anti-oxydant.

**Revendications pour les etats contractants: AT, ES, GR**

1. Procédé de préparation d'un composé bicyclique aromatique répondant à la formule générale suivante:

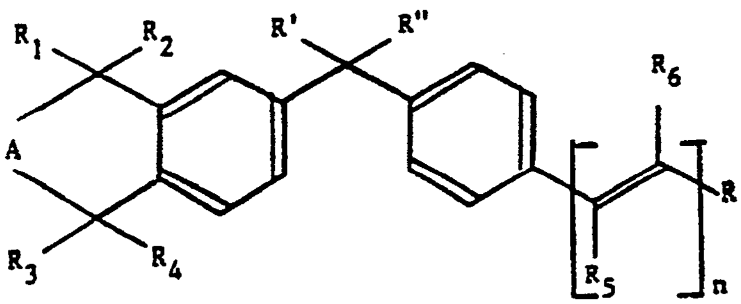

(I)

dans laquelle:

n est 0 ou 1,

R' représente un atome d'hydrogène, un radical OH, un radical alkoxy ayant de 1 à 4 atomes de carbone, ou un radical acyloxy ayant de 1 à 4 atomes de carbone,

R" représente un atome d'hydrogène ou un radical alkoxy ayant de 1 à 4 atomes de carbone,

ou R' er R", pris ensemble forment un radical oxo (=0), méthano (=$CH_2$) ou hydroxyimino (=N-OH),

R représente le radical -$CH_2OH$ ou le radical -$COR_7$,

$R_7$ représentant un atome d'hydrogène, le radical -$OR_8$ ou le radical

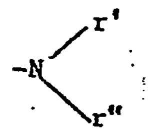

$R_8$ représentant un atome d'hydrogène, un radical alkyle ayant de 1 à 20 atomes de carbone, monohydroxyalkyle, polyhydroxyalkyle, aryle ou aralkyle éventuellement substitué(s) ou un reste d'un sucre ou encore le radical

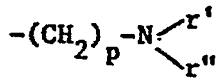

p étant 1, 2 ou 3 et r' et r" représentant un atome d'hydrogène, un radical alkyle inférieur, un radical monohydroxyalkyle éventuellement interrompu par un hétéroatome, un radical polyhydroxyalkyle, un radical aryle ou benzyle éventuellement substitué(s), un reste d'aminoacide ou de sucre aminé ou pris ensemble forment avec l'atome d'azote auquel ils sont rattachés, un hétérocycle,
A représente un radical méthylène ou diméthylène, substitué ou non, par un radical alkyle inférieur,
$R_1$, $R_2$, $R_3$ et $R_4$ représentent un atome d'hydrogène ou un radical alkyle inférieur,
$R_1$ et $R_3$, pris ensemble, pouvant former un radical méthylène ou diméthylène, lorsque A représente un radical diméthylène,
$R_5$ et $R_6$ représentent un atome d'hydrogène ou un radical méthyle, caractérisé par le fait qu'il consiste à faire réagir en milieu solvant organique un halogénure tel qu'un chlorure d'acide correspondant à la formule suivante:

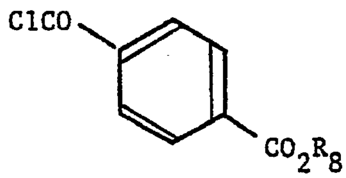

(1)

sur un composé aromatique correspondant à l'une des formules suivantes:

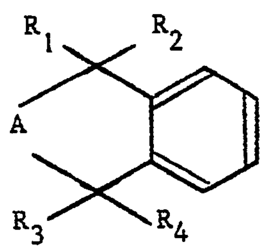

(2)

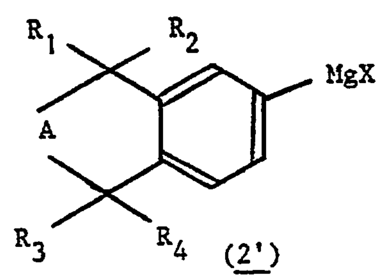

(2')

dans lesquels:
A, $R_1$ à $R_4$ ont les mêmes significations que celles données ci-dessus, $R_8$ étant un radical alkyle ayant de 1 à 20 atomes de carbone, et X est Br ou Cl, que l'on procède, si nécessaire à la saponification du céto-ester obtenu en céto-acide correspondant et à la transformation subséquente dudit céto-acide en amide correspondant par action d'une amine de formule:

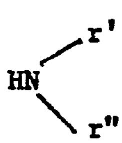

dans laquelle:
r' et r" ont les mêmes significations que celles données ci-dessus, ou à la transformation subséquente dudit céto-acide en hydroxyacide ou en diol et à l'oxydation éventuelle du diol en céto-aldéhyde correspondant.

2. Procédé selon la revendication 1, caractérisé par le fait que la réaction de condensation, avec le composé aromatique (2) est effectuée en pésence de chlorure d'aluminium anhydre dans du dichloro-1,2 éthane à une température comprise entre 0 et 25°C sous agitation.

3. Procédé selon la revendication 1, caractérisé par le fait que la condensation du chlorure d'acide sur l'organo magnésien (2') est effectuée dans le THF à une température d'environ 0°C.

4. Procédé selon la revendication 1, caractérisé par le fait que la préparation de l'amide est effectuée en présence de N,N'-carbonyl diimidazole.

5. Procédé selon la revendication 1, caractérisé par le fait que la réduction du céto-acide en hydroxy-acide correspondant est effectuée en présence de borohydrure de sodium dans le T.H.F.

6. Procédé selon la revendication 1, caractérisé par le fait que le céto-aldéhyde est obtenu par oxydation du diol à l'aide de chlorochromate de pyridinium, ledit diol correspondant résultant d'une réaction de réduction du céto-acide en présence d'hydrure de lithium aluminium.

7. Procédé de préparation d'un composé de formule:

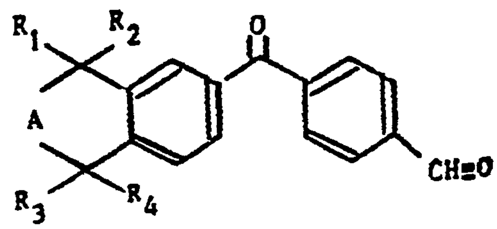

caractérisé par le fait qu'il consiste à faire réagir en milieu solvant organique dans les conditions de la réaction de Friedel-Crafts un chlorure d'acide de formule suivante:

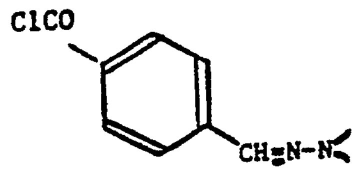

sur un composé aromatique de formule:

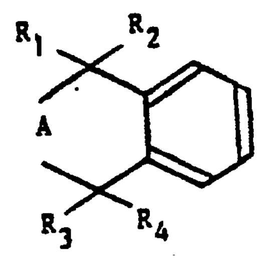

et que l'on procède ensuite à la libération de la fonction aldéhyde par échange avec le glyoxal pour conduire au céto-aldéhyde attendu.

8. Procédé de préparation d'un composé de formule:

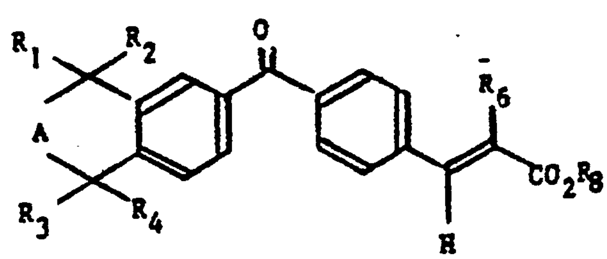

dans laquelle:

$R_1$ à $R_4$, $R_6$ et $R_8$ ayant les mêmes significations qu'à la revendication 1, caractérisé par le fait que l'on fait réagir un céto-aldéhyde tel qu'obtenu aux revendications 1 à 7 et ayant la formule suivante :

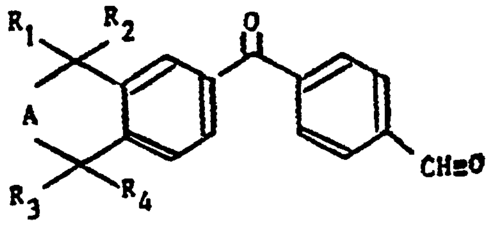

dans laquelle:
A et $R_1$1 à $R_4$ ont les mêmes significations qu'à la revendication 1, avec un phosphonoacétate d'alkyle de formule:

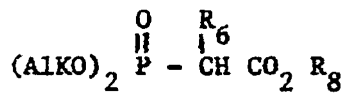

en présence d'hydrure de sodium dans le T.H.F. et que l'on soumet le céto-ester insaturé obtenu aux conditions conventionnelles de réaction permettant d'accéder aux différentes significations des radicaux de la formule (I) selon la revendication 1.

9. Utilisation d'un composé obtenu selon l'une quelconque des revendications 1 à 8 pour la préparation d'une composition pharmaceutique destinée au traitement des affections dermatologiques, respiratoires ainsi qu'ophtalmologiques.

**Patentansprüche für die Vertragsstaaten: BE, CH, DE, FR, GB, IT, LI, NL, SE**

1. Bicyclische aromatische Verbindungen der allgemeinen Formel:

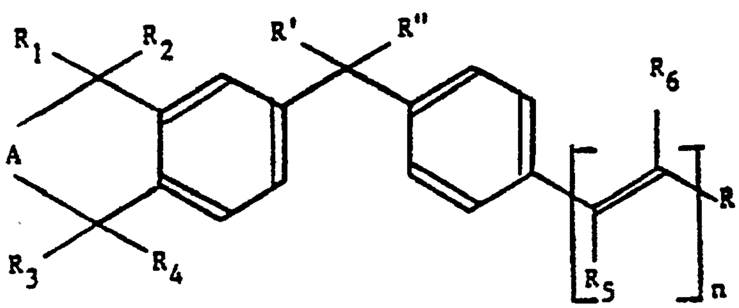

(I)

worin
n entweder 0 oder 1 bedeutet,
R' ein Wasserstoffatom, einen OH-Rest, einen Alkoxyrest mit 1 bis 4 Kohlenstoffatomen oder einen Acyloxyrest mit 1 bis 4 Kohlenstoffatomen bedeutet,
R" ein Wasserstoffatom oder einen Alkoxyrest mit 1 bis 4 Kohlenstoffatomen bedeutet, oder
R' und R" zusammen einen Oxo(=O)-, Methano (=$CH_2$)-, oder Hydroxyimino (=N-OH)-Rest bedeuten,
R einen $CH_2OH$- oder einen -$COR_7$-Rest darstellt,
$R_7$ ein Wasserstoffatom, einen -$OR_8$-Rest oder den Rest

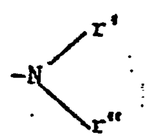

bedeutet,
$R_8$ ein Wasserstoffatom, einen Alkylrest mit 1 bis 20 Kohlenstoffatomen, einen Monohydroxyalkyl-, Polyhydroxyalkyl-, einen gegebenenfalls substituierten Aryl- oder Aralkylrest, einen Zuckerrest oder einen Rest der Formel:

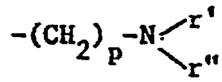

darstellt, worin
p für 1, 2 oder 3 steht und
r' und r" ein Wasserstoffatom, einen Niedrigalkyl-, einen Monohydroxyalkylrest, der gegebenenfalls durch ein Heteroatom unterbrochen ist, einen Polyhydroxyalkylrest, einen gegebenenfalls substituierten Aryl- oder Benzylrest, einen Aminosäurerest oder den Rest eines aminierten Zuckers oder zusammen mit dem Stickstoffatom, an welches sie gebunden sind, einen Heterocyclus bedeuten,
A einen Methylen- oder Dimethylenrest bedeutet, welcher unsubstituiert oder mit einem Niedrigalkylrest substituiert ist,
$R_1$, $R_2$, $R_3$ und $R_4$ ein Wasserstoffatom oder einen Niedrigalkylrest bedeuten,
$R_1$ und $R_3$ zusammen einen Methylen- oder Dimethylenrest bedeuten können, wenn A einen Dimethylenrest bedeutet,
$R_5$ und $R_6$ ein Wasserstoffatom oder einen Methylrest bedeuten und die Salze dieser Verbindungen sowie ihre optischen und geometrischen Isomere.

2. Verbindungen nach Anspruch 1, dadurch gekennzeichnet, daß die Niedrigalkylreste und diejenigen, die bis zu 20 Kohlenstoffatome haben, ausgewählt sind aus der Gruppe, bestehend aus Methyl, Ethyl, Isopropyl, Butyl, tert.-Butyl, 2-Ethylhexyl, Isooctyl, Dodecyl, Hexadecyl oder Octadecyl.

3. Verbindungen nach Anspruch 1, dadurch gekennzeichnet, daß der Monohydroxyalkylrest ein 2-Hydroxyethyl-, 2-Hydroxypropyl- oder einen 2-Hydroxyethoxyethylrest ist.

4. Verbindungen nach Anspruch 1, dadurch gekennzeichnet, daß der Polyhydroxyalkylrest ein 2,3-Dihydroxypropyl-,1,3-Dihydroxy-2-propyl- oder Pentaerythritrest ist.

5. Verbindungen nach Anspruch 1, dadurch gekennzeichnet, daß der Alkoxyrest ein Methoxy-, Isopropoxy-, Butoxy- oder tert.-Butoxyrest ist.

6. Verbindungen nach Anspruch 1, dadurch gekennzeichnet, daß der Arylrest ein Phenylrest ist, welcher gegebenenfalls durch ein Halogenatom, einen OH-, $NO_2$-, Niedrigalkyl-, Trifluormethyl- oder Carbonsäurerest substituiert ist.

7. Verbindungen nach Anspruch 1, dadurch gekennzeichnet, daß der Aralkylrest ein Benzyl- oder Phenethylrest ist.

8. Verbindungen nach Anspruch 1, dadurch gekennzeichnet, daß der Zuckerrest ein von Glucose, Mannose, Erythrose oder Galactose abgeleiteter Rest ist.

9. Verbindungen nach Anspruch 1, dadurch gekennzeichnet, daß der aminierte Zuckerrest ein von Glucosamin, Galactosamin, Mannosamin oder Meglumin abgeleiteter Rest ist.

10. Verbindungen nach Anspruch 1, dadurch gekennzeichnet, daß die Reste r' und r" zusammen mit dem Stickstoffatom, an welches sie gebunden sind, einen Piperidino-, Piperazino-, Morpholino-, Pyrrolidino- oder 4-(2-Hydroxyethyl)piperazinorest bedeuten.

11. Verbindungen nach einem der Ansprüche 1 bis 9 der allgemeinen Formel:

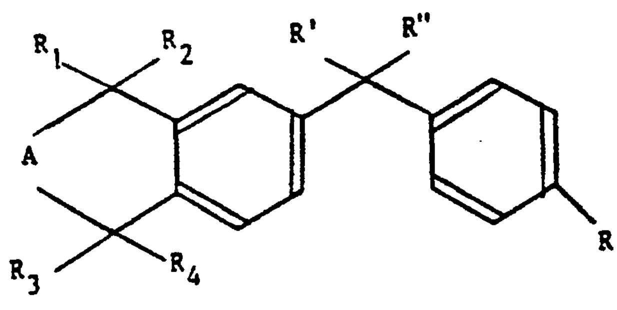

(II)

worin
A, R, $R_1$ bis $R_4$, R' und R" die in Anspruch 1 angegebenen Bedeutungen besitzen.

12. Verbindungen nach einem der Ansprüche 1 bis 9 der allgemeinen Formel:

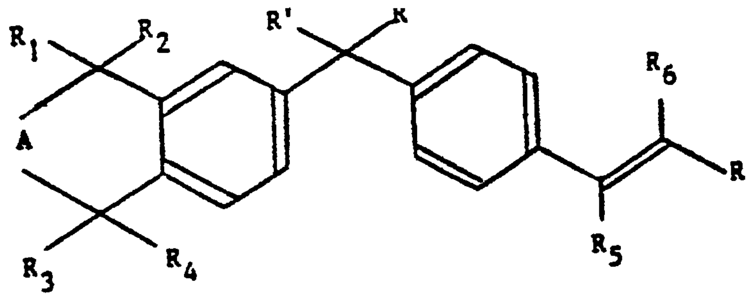

(III)

worin
A, R, $R_1$ bis $R_6$, R' und R" die in Anspruch 1 angegebenen Bedeutungen besitzen.

13. Verbindungen nach einem der Ansprüche 1 bis 10 der allgemeinen Formel:

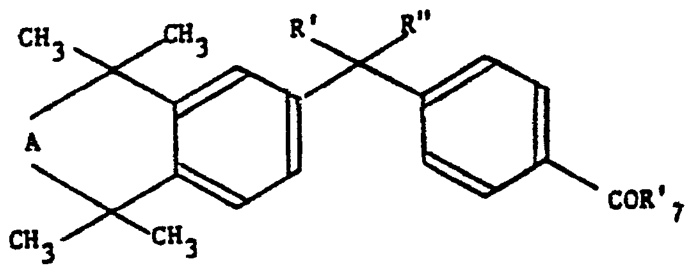

(IV)

worin
A für

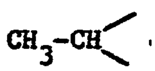

oder einen Dimethylenrest steht,
R' einen OH-Rest und R" ein Wasserstoffatom oder R' und R" zusammen einen Oxorest (=O) bedeuten, und $R'_7$ ein Wasserstoffatom, einen $OR'_8$-Rest oder

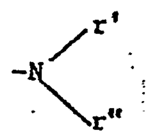

bedeutet,
$R'_8$ ein Wasserstoffatom oder einen Niedrigalkylrest bedeutet,
r' ein Wasserstoffatom bedeutet und
r" einen Niedrigalkylrest oder einen substituierten Phenylrest bedeutet, oder
r' und r" zusammen mit dem Stickstoffatom, an welches sie gebunden sind, einen 4-(2-Hydroxyethyl)piperazinorest bedeuten.

14. Verbindungen nach einem der Ansprüche 1 bis 9 oder nach Anspruch 11, der allgemeinen Formel:

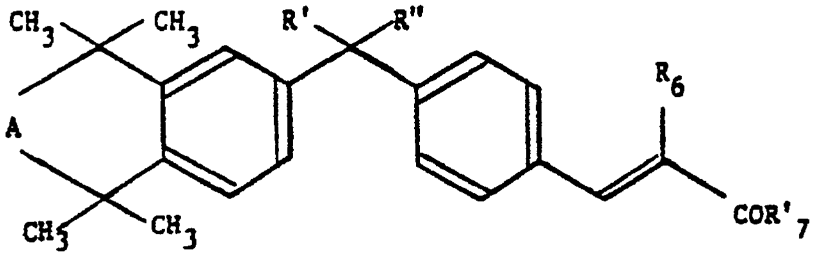

(V)

worin

A einen

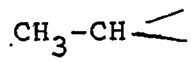

oder einen Dimethylenrest bedeutet,
R' einen Hydroxyrest und
R" ein Wasserstoffatom bedeuten oder
R' und R" zusammen einen Oxorest (=O) bedeuten,
$R_6$ ein Wasserstoffatom oder einen Methylrest bedeutet, und
$R'_7$ einen $OR'_8$-Rest oder

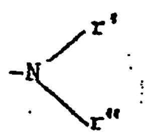

bedeutet,
$R'_8$ ein Wasserstoffatom oder einen Niedrigalkylrest bedeutet,
r' ein Wasserstoffatom und
r" einen Niedrigalkylrest bedeuten.

15. Verbindungen nach einem der Ansprüche 1 bis 13, ausgewählt aus der Gruppe folgender Verbindungen:
(1) Methyl-4-(5,5,8 8-tetramethyl-5,6,7,8-tetrahydro-2-naphthyl)carbonylbenzoat
(2) 4-(5,5,8,8-Tetramethyl-5,6,7,8-tetrahydro-2-naphthyl)carbonylbenzoesäure
(3) 4-(5,8-Methano-5,6,7,8-tetrahydro-2-naphthyl)carbonylbenzaldehyd
(4) 4-(5,5,8,8-Tetramethyl-5,6,7,8-tetrahydro-2-naphthyl)carbonylbenzaldehyd
(5) Methyl-4-(1,1,2,3,3-pentamethyl-5-indanyl)carbonylbenzoat
(6) 4-(1,1,2,3,3-Pentamethyl-5-indanyl)carbonylbenzoesäure
(7) 4-(1,1,2,3,3-Pentamethyl-5-indanyl)carbonylbenzaldehyd
(8) 1-(1,1,2,3,3-Pentamethyl-5-indanyl)-1-(4-hydroxymethyl-phenyl)methanol,
(9) 4-(5,5,8,8-Tetramethyl-5,6,7,8-tetrahydro-2-naphthyl)-hydroxymethylbenzoesäure,
(10) 4-(1,1,3,3-Pentamethyl-5-indanyl)hydroxymethylbenzoesäure
(11) Methyl-4-(1,1,2,3,3-pentamethyl-5-indanyl)hydroxybenzoat
(12) 4-(5,5,8,8-Tetramethyl-5,6,7,8-tetrahydro-2-naphthyl)carbonyl-N-ethylbenzamid
(13) 4-(1,1,2,3,3-Pentamethyl-5-indanyl)carbonyl-N-ethylbenzamid
(14) trans-Ethyl-4-(5,8-methano-5,6,7,8-tetrahydro-2-naphthyl) carbonylcinnamat
(15) trans-4-(5,8-Methano-5,6,7,8-tetrahydro-2-napthyl)carbonylzimtsäure
(16) trans-Ethyl-4-(5,5,8,8-tetramethyl-5,6,7,8-tetrahydro-2-naphthyl)carbonylcinnamat
(17) trans-4-(5,5,8,8-Tetramethyl-5,6,7,8-tetrahydro-2-naphthyl)carbonylzimtsäure
(18) trans-Ethyl-4-(5,5,8,8-tetramethyl-,5,6,7,8-tetrahydro-2-naphthyl)carbonyl-α-methylcinnamat
(19) trans-4-(5,5,8,8-Tetramethyl-5,6,7,8-tetrahydro-2-naphthyl )carbonyl-α-methylzimtsäure
(20) trans-Ethyl-4-(1,1,2,3,3-pentamethyl-5-indanyl)-carbonyl-α-methylcinnamat
(21) trans-4-(1,1,2,3,3-Pentamethyl-5-indanyl)carbonyl-α-methylzimtsäure
(22) trans-Ethyl-4-(5,5,8,8-tetramethyl-5,6,7,8-tetrahydro-2-naphthyl)hydroxymethyl-α-methylcinnamat
(23) trans-4-(5,5,8,8-Tetramethyl-5,6,7,8-tetrahydro-2-naphthyl)hydroxymethyl-α-methylzimtsäure
(24) trans-4-(5,5,8,8-Tetramethyl-5,6,7,8-tetrahydro-2-naphthyl)hydroxymethyl-α-methyl-N-ethylcinnamid
(25) trans-4-(5,5,8,8-Tetramethyl-5,6,7,8-tetrahydro-2-naphthyl)carbonyl-α-methyl-N-ethylcinnamid
(26) trans-4-(1,1,2,3,3-Pentamethyl-5-indanyl)carbonyl-α-methyl-N-ethylcinnamid
(27) trans-Ethyl-5-(5,5,8,8-tetramethyl-5,6,7,8-tetrahydro-2-naphthyl)hydroxymethylcinnamat
(28) trans-4-(5,5,8,8-Tetramethyl-5,6,7,8-tetrahydro-2-napthyl)hydroxymethylzimtsäure
(29) 4'-(2-Hydroxyethyl)-piperazino-4-(5,5,8,8-tetramethyl-5,6,7,8-tetrahydro-2-naphthyl) carbonylbenzamid
(30) N-(3,5-Ditrifluormethyl-1-phenyl)-4-(5,5,8,8-tetramethyl-5,6,7,8-tetrahydro-2-naphthyl) carbonylbenzamid.

16. Verfahren zur Herstellung von Verbindungen der Ansprüche 1 bis 15, dadurch gekennzeichnet, daß man in einem organischen Lösungsmittel ein Halogenid, z.B. ein Säurehalogenid der allgemeinen Formel

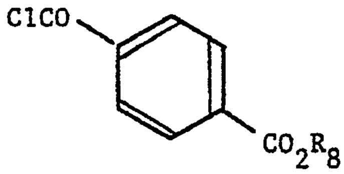

(1)

mit einer aromatischen Verbindung einer der allgemeinen Formeln:

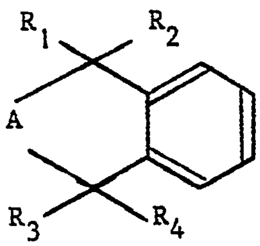

(2)

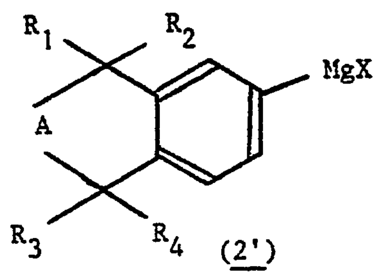

(2')

umsetzt, worin
A, $R_1$ bis $R_4$ die in Anspruch 1 angegebenen Definitionen besitzen, $R_8$ einen Alkylrest mit 1 bis 20 Kohlenstoffatomen bedeutet und X ein Brom- oder Chloratom bedeutet, daß man, wenn nötig, den erhaltenen Ketoester zur korrespondierenden Ketosäure verseift und anschließend die Ketosäure durch Umsetzung mit einem Amin der allgemeinen Formel

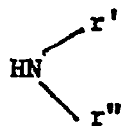

worin
r' und r" den in Anspruch 1 angegebenen Definitionen entsprechen, in das korrespondierende Amid überführt oder die Ketosäure anschließend in die Hydroxysäure oder in das Diol überführt und gegebenenfalls das Diol durch Oxidation in den entsprechenden Ketoaldehyd überführt.

17. Verfahren nach Anspruch 16, dadurch gekennzeichnet, daß die Kondensationsreaktion mit der aromatischen Verbindung (2) in Gegenwart von wasserfreiem Aluminiumchlorid in 1,2-Dichlorethan, in einem Temperaturbereich zwischen 0 und 25°C und unter Rühren durchgeführt wird.

18. Verfahren nach Anspruch 16, dadurch gekennzeichnet, daß die Kondensation des Säurechlorids mit der Organomagnesiumverbindung (2') in THF und bei einer Temperatur von etwa 0°C durchgeführt wird.

19. Verfahren nach Anspruch 16, dadurch gekennzeichnet, daß die Herstellung des Amids in Gegenwart von N,N'-Carbonyldiimidazol durchgeführt wird.

20. Verfahren nach Anspruch 16, dadurch gekennzeichnet, daß die Reduktion der Ketosäure zur korrespondierenden Hydroxysäure in Gegenwart von Natriumborhydrid in THF durchgeführt wird.

21. Verfahren nach Anspruch 16, dadurch gekennzeichnet, daß der Ketoaldehyd durch Oxidation des Diols mit Hilfe von Pyridiniumchlorochromat erhalten wird, wobei das entsprechende Diol durch Reduktion der Ketosäure in Gegenwart von Lithiumaluminiumhydrid entsteht.

22. Verfahren zur Herstellung von Verbindungen der allgemeinen Formel (I), worin n=O und R=CHO bedeutet, dadurch gekennzeichnet, daß man in einem organischen Lösungsmittel unter Bedingungen der Friedel-Crafts-Reaktion ein Säurechlorid der allgemeinen Formel:

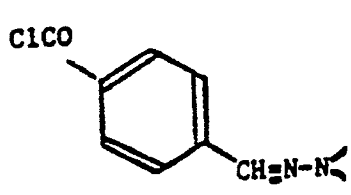

mit einer aromatischen Verbindung der allgemeinen Formel:

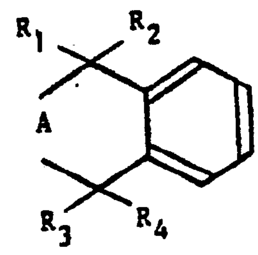

umsetzt, und daß man anschließend durch Austausch mit Glyoxal die Aldehydfunktion freisetzt, wodurch man den gewünschten Ketoaldehyd erhält.

23. Verfahren zur Herstellung von Verbindungen der allgemeinen Formel:

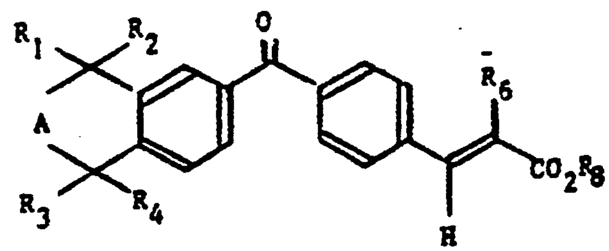

worin

$R_1$ bis $R_4$, $R_6$ und $R_8$ die in Anspruch 1 angegebenen Bedeutungen besitzen, dadurch gekennzeichnet, daß man einen nach Anspruch 16 oder 22 hergestellten Ketoaldehyd der allgemeinen Formel:

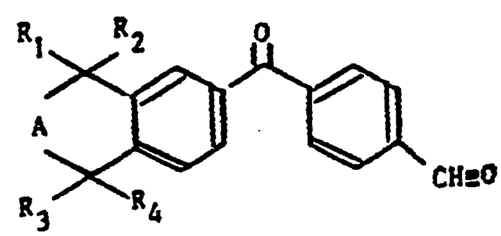

worin A und $R_1$ bis $R_4$ den in Anspruch 1 angegebenen Definitionen entsprechen, mit einem Alkylphosphonoacetat der allgemeinen Formel

$$(\mathrm{AlKO})_2\overset{\overset{\mathrm{O}}{\|}}{\mathrm{P}} - \overset{\overset{R_6}{|}}{\mathrm{CH}}\ \mathrm{CO}_2\ R_8$$

in Gegenwart von Natriumhydrid in THF umsetzt, und den erhaltenen ungesättigten Ketoester herkömmlichen Reaktionen unterzieht, um Verbindungen der Formel (I) nach Anspruch 1 mit unterschiedlichen Resten zu erhalten.

24. Arzneimittel, dadurch gekennzeichnet, daß es eine Verbindung der allgemeinen Formel (I) nach einem der Ansprüche 1 bis 15 oder hergestellt nach einem der Ansprüche 16 bis 23, darstellt.

25. Arzneimittel nach Anspruch 24, dadurch gekennzeichnet, es in einer täglichen Dosis von etwa 2 µg bis 2 mg pro kg Körpergewicht verabreicht wird.

26. Pharmazeutische Mittel, dadurch gekennzeichnet, daß sie in einem geeigneten Träger, für eine enterale, parenterale, topische oder okulare Verabreichung, mindestens eine Verbindung der allgemeinen Formel (I) nach einem der Ansprüche 1 bis 15, oder hergestellt nach einem der Ansprüche 16 bis 23, enthalten.

27. Mittel nach Anspruch 26, dadurch gekennzeichnet, daß sie in einer geeigneten Form für eine topische oder okulare Anwendung vorliegen und 0,0005 bis etwa 5 Gew.-% einer Verbindung der allgemeinen Formel (I) enthalten.

28. Verwendung einer Verbindung nach einem der Ansprüche 1 bis 15 oder einer nach einem der Ansprüche 16 bis 23 hergestellten Verbindung zur Herstellung eines pharmazeutisohen Mittels zur Behandlung von dermatologischen, respiratorischen sowie ophtalmologischen Erkrankungen.

29. Kosmetische Mittel zur Körper- und Haarpflege, dadurch gekennzeichnet, daß sie in einem geeigneten kosmetischen Träger mindestens eine Verbindung der allgemeinen Formel (I) nach einem der Ansprüche 1 bis 15 oder hergestellt nach einem der Ansprüche 16 bis 23 enthalten.

30. Kosmetische Mittel nach Anspruch 29, dadurch gekennzeichnet, daß sie die Verbindung der allgemeinen Formel (I) in einem Konzentrationsbereich von 0,0005 bis 2% und vorzugsweise von 0,01 bis 1 Gew.-% enthalten.

31. Mittel nach einem der Ansprüche 26 oder 29, dadurch gekennzeichnet, daß sie außerdem mindestens ein inertes Additiv oder ein pharmakodynamisch oder kosmetisch aktives Additiv, z.B. ein Hydratisierungsmittel, ein Anti-Seborrhoemittel, ein Anti-Aknemittel, ein Antibiotikum, ein Mittel, das das Nachwachsen der Haare fördert, ein antiinflammatorisches Mittel, ein Carotinoid, ein Antipsoriasis-Mittel, einen Geschmacksstoff, ein Konservierungsmittel, ein Stabilisierungsmittel, ein feuchtigkeits-regulierendes Mittel, ein pH-regulierendes Mittel, ein Mittel zur Modifikation des osmotischen Druckes, ein emulgierendes Mittel, einen UV-A- oder UV-B-Filter oder ein Antioxidans enthalten.

**Patentansprüche für die Vertragsstaaten: AT, ES, GR**

1. Verfahren zur Herstellung einer bicyclischen aromatischen Verbindung der allgemeinen Formel:

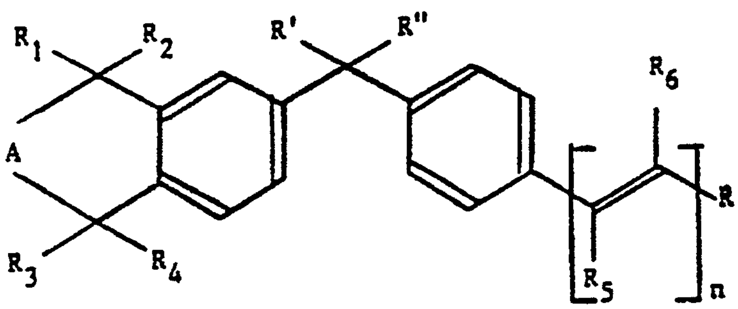

(I)

worin
n entweder 0 oder 1 bedeutet,
R′ ein Wasserstoffatom, einen OH-Rest, einen Alkoxyrest mit 1 bis 4 Kohlenstoffatomen oder einen Acyloxyrest mit 1 bis 4 Kohlenstoffatomen bedeutet,
R″ ein Wasserstoffatom oder einen Alkoxyrest mit 1 bis 4 Kohlenstoffatomen bedeutet, oder
R′ und R″ zusammen einen Oxo(=O)–, Methano (=$CH_2$)–, oder Hydroxyimino (=N–OH)-Rest bedeuten,
R einen $CH_2OH$- oder einen $-COR_7$-Rest darstellt,
$R_7$ ein Wasserstoffatom, einen $-OR_8$-Rest oder den Rest

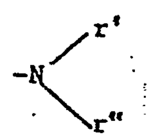

bedeutet,
$R_8$ ein Wasserstoffatom, einen Alkylrest mit 1 bis 20 Kohlenstoffatomen, einen Monohydroxyalkyl-, Polyhydroxyalkyl-, einen gegebenenfalls substituierten Aryl- oder Aralkylrest, einen Zuckerrest oder einen Rest der Formel:

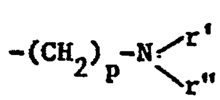

darstellt, worin
p für 1, 2 oder 3 steht und
r′ und r″ ein Wasserstoffatom, einen Niedrigalkyl-, einen Monohydroxyalkylrest, der gegebenenfalls durch ein Heteroatom unterbrochen ist, einen Polyhydroxyalkylrest, einen gegebenenfalls substituierten Aryl- oder Benzylrest, einen Aminosäurerest oder den Rest eines aminierten Zuckers oder zusammen mit dem Stickstoffatom, an welches sie gebunden sind, einen Heterocyclus bedeuten,
A einen Methylen- oder Dimethylenrest bedeutet, welcher unsubstituiert oder mit einem Niedrigalkylrest substituiert ist,
$R_1$, $R_2$, $R_3$ und $R_4$ ein Wasserstoffatom oder einen Niedrigalkylrest bedeuten,

$R_1$ und $R_3$ zusammen einen Methylen- oder Dimethylenrest bedeuten können, wenn A einen Dimethylenrest bedeutet,

$R_5$ und $R_6$ ein Wasserstoffatom oder einen Methylrest bedeuten,

dadurch gekennzeichnet, daß man in einem organischen Lösungsmittel ein Halogenid, z.B. ein Säurehalogenid der allgemeinen Formel:

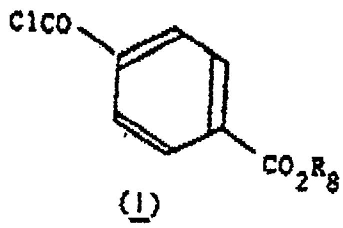

(1)

mit einer aromatischen Verbindung einer der allgemeinen Formeln:

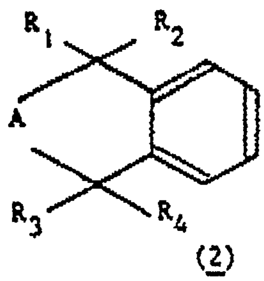

(2)

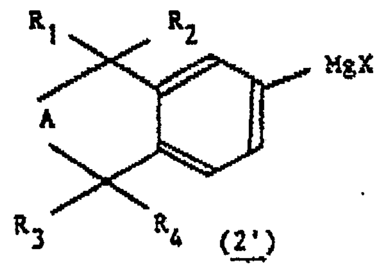

(2')

umsetzt,

worin

A, $R_1$ bis $R_4$ die in Anspruch 1 angegebenen Definitionen besitzen, $R_8$ einen Alkylrest mit 1 bis 20 Kohlenstoffatomen bedeutet und X ein Brom- oder Chloratom bedeutet,

daß man, wenn nötig, den erhaltenen Ketoester zur korrespondierenden Ketosäure verseift und anschließend die Ketosäure durch Umsetzung mit einem Amin der allgemeinen Formel

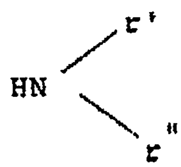

worin

r' und r'' den in Anspruch 1 angegebenen Definitionen entsprechen,

in das korrespondierende Amid überführt oder die Ketosäure anschließend in die Hydroxysäure oder in das Diol überführt und gegebenenfalls das Diol durch Oxidation in den entsprechenden Ketoaldehyd überführt.

2. Verfahren nach Anspruch 1, dadurch gekennzeichnet, daß die Kondenstaionsreaktion mit der aromatischen Verbindung (2) in Gegenwart von wasserfreiem Aluminiumchlorid in 1,2-Dichlorethan, in einem Temperaturbereich zwischen 0 und 25°C und unter Rühren durchgeführt wird.

3. Verfahren nach Anspruch 1, dadurch gekennzeichnet, daß die Kondensation des Säurechlorids mit der Organomagnesiumverbindung (2') in THF und bei einer Temperatur von etwa 0°C durchgeführt wird.

4. Verfahren nach Anspruch 1, dadurch gekennzeichnet, daß die Herstellung des Amids in Gegenwart von N,N'-Carbonyldiimidazol durchgeführt wird.

5. Verfahren nach Anspruch 1, dadurch gekennzeichnet, daß die Reduktion der Ketosäure zur korrespondierenden Hydroxysäure in Gegenwart von Natriumborhydrid in TMF durchgeführt wird.

6. Verfahren nach Anspruch 1, dadurch gekennzeichnet, daß der Ketoaldehyd durch Oxidation des Diols mit Hilfe von Pyridioniumchlorochromat erhalten wird, wobei das entsprechende Diol durch Reduktion der Ketosärue in Gegenwart von Lithiumaluminiumhydrid entsteht.

7. Verfahren zur Herstellung einer Verbindung der Formel (I)

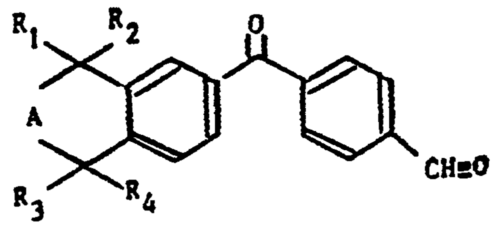

dadurch gekennzeichnet, daß man in einem organischen Lösungsmittel unter Bedingungen der Friedel-Crafts-Reaktion ein Säurechlorid der allgemeinen Formel:

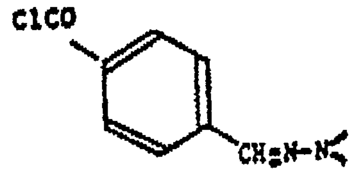

mit einer aromatischen Verbindung der allgemeinen Formel:

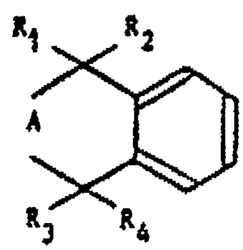

umsetzt, und daß man anschließend durch Austausch mit Glyoxal die Aldehydfunktion freisetzt, wodurch man den gewünschten Ketoaldehyd erhält.

8. Verfahren zur Herstellung einer Verbindung der Formel

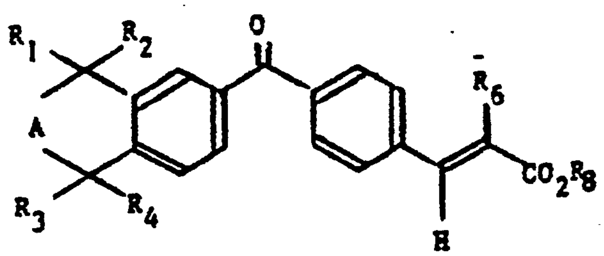

worin
$R_1$ bis $R_4$, $R_6$ und $R_8$ die in Anspruch 1 angegebenen Bedeutungen besitzen,
dadurch gekennzeichnet, daß man einen nach den Ansprüchen 1 bis 7 erhaltenen Ketoaldehyd der allgemeinen Formel:

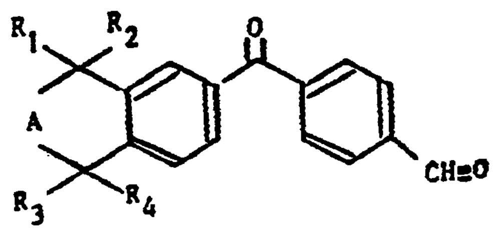

worin
A und $R_1$ bis $R_4$ den in Anspruch 1 angegebenen Definitionen entsprechen,
mit einem Alkylphosphonoacetat der allgemeinen Formel

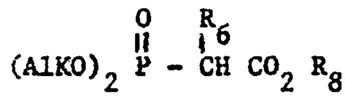

in Gegenwart von Natriumhydrid in THF umsetzt, und den erhaltenen ungesättigten Ketoester herkommlichen Reaktionen unterzieht, um Verbindungen der Formel (I) nach Anspruch 1 mit unterschiedlichen Resten zu erhalten.

9. Verwendung einer nach einem der Ansprüche 1 bis 8 erhaltenen Verbindung zur Herstellung eines pharmazeutischen Mittels zur Behandlung von dermatologischen, respiratorischen sowie ophtalmologischen Erkrankungen.

**Claims for the contracting states: BE, CH, DE, FR, GB, IT, LI, NL, SE**

1. Aromatic bicyclic compounds, characterized in that they correspond to the following formula:

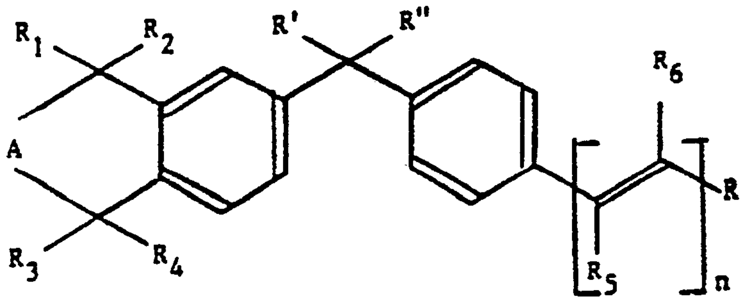
(I)

in which:
n is 0 or 1,
R′ represents a hyydrogen atom, an OH radical, an alkoxy radical having from 1 to 4 carbon atoms or an acyloxy radical having from 1 to 4 carbon atoms,
R″ represents a hydrogen atom or an alkoxy radical having from 1 to 4 carbon atoms,
or R′ and R″, taken together, form an oxo (=O), methano (=$CH_2$) or hydroxyimino (=N–OH) radical,
R represents a –$CH_2OH$ radical or a radical –$COR_7$,
R""$_7$representing a hydropen atom a radical -$OR_8$or a radical

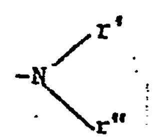

$R_8$ representing a hydrogen atom, an alkyl radical having from 1 to 20 carbon atoms, a monohydroxyalkyl, polyhydroxyalkyl or optionally substituted aryl or aralkyl radical or a sugar residue, or alternatively a radical

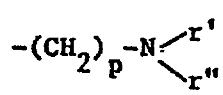

p being 1, 2 or 3 and r′ and r″ representing a hydrogen atom, a lower alkyl radical, a monohydroxyalkyl radical optionally interrupted by a hetero atom, a polyhydroxyalkyl radical, an optionally substituted aryl or benzyl radical, an amino acid residue or an amino sugar residue or, taken together with the nitrogen atom to which they are attached form a heterocycle,
A represents a methylene or dimethylene radical, unsubstituted or substituted with a lower alkyl radical
$R_1$, $R_2$, $R_3$ and $R_4$ represent a hydrogen atom or a lower alkyl radical,
it being possible for $R_1$ and $R_3$, taken together, to form a methylene or dimethylene radical when A represents a dimethylene radical, and
$R_5$ and $R_6$ represent a hydrogen atom or a methyl radical,
and the salts of the said compounds as well as their optical and geometrical isomers.

2. Compounds according to Claim 1, characterized in that the lower alkyl radicals and those having up to 20 carbon atoms are selected from the group consisting of methyl, ethyl, isopropyl, butyl, tert-butyl, 2-ethyl-hexyl, isooctyl, dodecyl, hexadecyl and octadecyl radicals.

3. Compounds according to Claim 1, characterized in that the monohydroxyalkyl radical is a 2-hydroxyethyl, 2-hydroxypropyl or 2-hydroxyethoxyethyl radical.

4. Compounds according to Claim 1, characterized in that the polyhydroxyalkyl radical is a 2,3-dihydroxypropyl or 1,3-dihydroxy-2-propyl radical or a pentaerythritol residue.

5. Compounds according to Claim 1, characterized in that the alkoxy radical is a methoxy, isopropoxy, butoxy or tert-butoxy radical.

6. Compounds according to Claim 1, characterized in that the aryl radical is a phenyl radical optionally substituted with a halogen atom, –OH, $-NO_2$, a lower alkyl radical, a trifluoromethyl radial or a carboxylic acid group.

7. Compounds according to Claim 1, characterized in that the aralkyl radical is a benzyl or phenethyl radical.

8. Compounds according to Claim 1, characterized in that the sugar residue is derived from glucose, mannose, erythrose or galactose.

9. Compounds according to Claim 1, characterized in that the amino sugar residue is derived from glucosamine, galactosamine, mannosamine or meglumine.

10. Compounds according to Claim 1, characterized in that the radicals r′ and r″, taken together with the nitrogen atom to which they are attached, form a piperidino, piperazino, morpholino, pyrrolidino or 4-(2-hydroxyethyl)piperazino radical.

11. Compounds according to any one of Claims 1 to 9, characterized in that they correspond to the following formula:

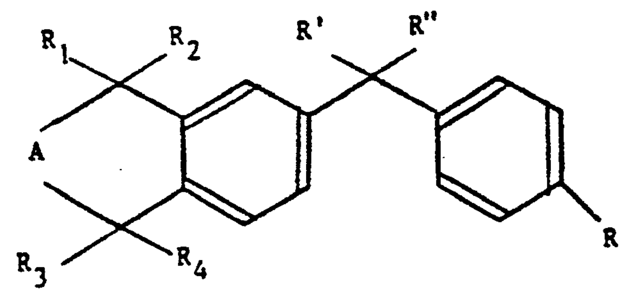

(II)

in which:
A, R, $R_1$ to $R_4$, R′ and R″ have the same meanings as those given in Claim 1.

12. Compounds according to any one of Claims 1 to 9, characterized in that they correspond to the following formula:

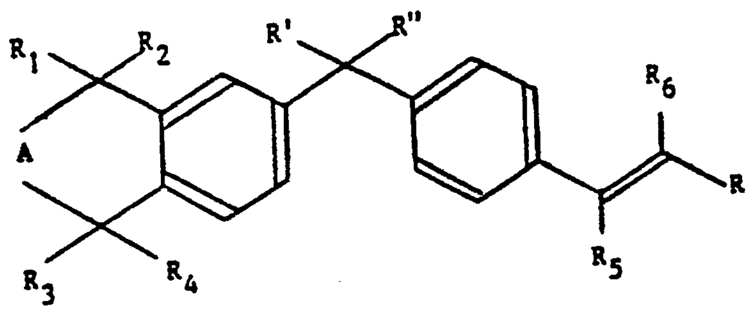

(III)

in which:
A, R, $R_1$ to $R_6$, R′ and R″ have the same meanings as those given in Claim 1.

13. Compounds according to any one of Claim 1 to 10, characterized in that they correspond to the following formula:

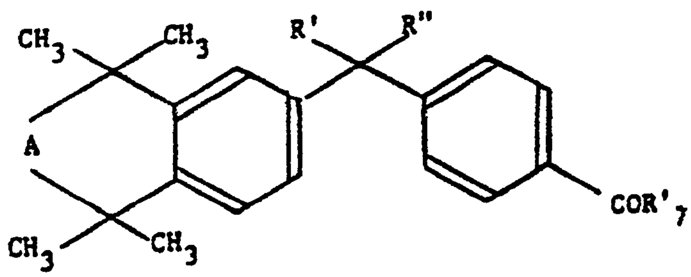

(IV)

in which:
A represents a

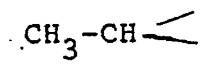

radical or a dimethylene radical,
R′ represents an OH radical and R″ represents a hydrogen atom, or R′ and R″, taken together, form an oxo (=O) radical,
and $R'_7$ represents a hydrogen atom, a radical $-OR'_8$

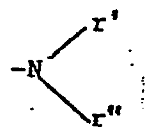

$R'_8$ representing a hydrogen atom or a lower alkyl radical,
r′ representing a hydrogen atom and r″ representing a lower alkyl radical or a substituted phenyl radical, or r′ and r″, taken together with the nitrogen atom to which they are attached, form a 4-(2-hydroxyethyl)piperazino radical.

14. Compounds according to any one of claims 1 to 9, or according to Claim 11, characterized in that they correspond to the following formula:

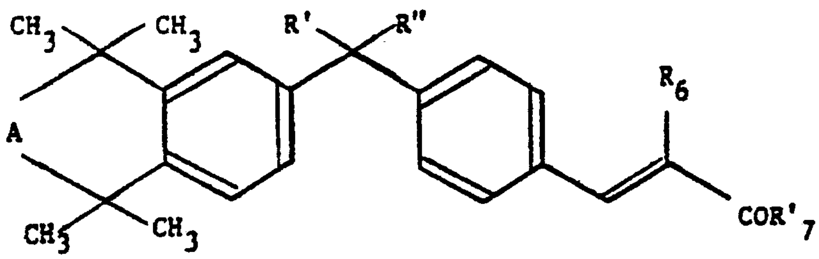

(V)

in which:
A represents a

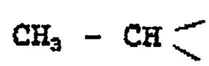

radical or a dimethylene radical,
R′ represents an OH radical and R″ a hydrogen atom, or R′ and R″, taken together, form an oxo (=O) radical,
$R_6$ represents a hydrogen atom or a methyl radical,
and $R'''_7$represents $-OR'_8$or a radical

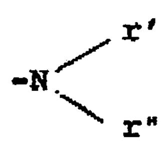

$R'_8$ representing a hydrogen atom or a lower alkyl radical,
r′ representing a hydrogen atom and r″ representing a lower alkyl radical.

15. Compounds according to any one of Claims 1 to 13, characterized in that they are selected from the group consisting of:
(1) methyl 4-[(5,5,8,8-tetramethyl-5,6,7,8-tetrahydro-2-naphthyl)carbonyl]benzoate,
(2) 4-[(5,5,8,8-tetramethyl-5,6,7,8-tetrahydro-2-naphthyl)carbonyl]benzoic acid,
(3) 4-[(5,8-methano-5,6,7,8-tetrahydro-2-naphthyl)carbonyl]benzaldehyde,
(4) 4-[(5,5,8,8-tetramethyl-5,6,7,8-tetrahydro-2-naphthyl)carbonyl]benzaldehyde,
(5) methyl 4-[(1,1,2,3,3-pentamethyl-5-indanyl)carbonyl]benzoate,
(6) 4-[(1,1,2,3,3,-pentamethyl-5-indanyl)-carbonyl]benzoic acid,
(7) 4-[(1,1,2,3,3-pentamethyl-5-indanyl)carbonyl]benzaldehyde,
(8) 1-(1,1,2,3,3,-pentamethyl-5-indanyl)-1-(4-hydroxymethylphenyl)methanol,
(9) 4-[(5,5,8,8-tetramethyl-5,6,7,8-tetrahydro-2-naphthyl)(hydroxy)methyl]benzoic acid,
(10) 4-[(1,1,2,3,3-pentamethyl-5-indanyl)(-hydroxy)methyl]benzoic acid,
(11) methyl 4-[(1,1,2,3,3-pentamethyl-5-indanyl)(hydroxy)methyl]benzoate,
(12) N-ethyl-4-[(5,5,8,8-tetramethyl-5,6,7,8-tetrahydro-2-naphthyl)carbonyl]benzamide,
(13) N-ethyl-4-[(1,1,2,3,3,-pentamethyl-5-indanyl)carbonyl]benzamide,
(14) ethyl trans-4-[(5,8-methano-5,6,7,8-tetrahydro-2-naphthyl)carbonyl]cinnamate,
(15) trans-4-[(5,8-methano-5,6,7,8-tetrahydro-2-naphthyl)carbonyl]cinnamic acid,
(16) ethyl trans-4-[(5,5,8,8-tetramethyl-5,6,7,8-tetrahydro-2-naphthyl)carbonyl]cinnamate,
(17) trans-4-[(5,5,8,8-tetramethyl-5,6,7,8-tetrahydro-2-naphthyl)carbonyl]cinnamic acid,
(18) ethyl trans-4-[(5,5,8,8-tetramethyl-5,6,7,8-tetrahydro-2-naphthyl)carbonyl]-$\alpha$-methyl-cinnamate,
(19) trans-4-[(5,5,8,8-tetramethyl-5,6,7,8-tetrahydro-2-naphthyl)carbonyl]-$\alpha$-methylcinnamic acid,
(20) ethyl trans-4-[(1,1,2,3,3-pentamethyl-5-indanyl)carbonyl]-$\alpha$-methylcinnamate,
(21) trans-4-[(1,1,2,3,3-pentamethyl-5-indanyl)carbonyl]-$\alpha$-methylcinnamic acid,
(22) ethyl trans-4-[(5,5,8,8-tetramethyl-5,6,7,8-tetrahydro-2-naphthyl)(hydroxy)methyl]-$\alpha$-methyl-cinnamate,
(23) trans-4-[(5,5,8,8-tetramethyl-5,6,7,8-tetrahydro-2-naphthyl)(hydroxy)methyl]-$\alpha$-methyl-cinnamic acid,
(24) N-ethyl-trans-4-[(5,5,8,8-tetramethyl-5,6,7,8-tetrahydro-2-naphthyl)(hydroxy)methyl]-$\alpha$-methyl-cinnamide,
(25) N-ethyl-trans-4-[(5,5,8,8-tetramethyl-5,6,7,8-tetrahydro-2-naphthyl)carbonyl]-$\alpha$-methyl-cinnamide,
(26) N-ethyl-trans-4-[(1,1,2,3,3-pentamethyl-5-indanyl)carbonyl]-$\alpha$-methylcinnamide,
(27) ethyl trans-4-[(5,5,8,8-tetramethyl-5,6,7,8-tetrahydro-2-naphthyl)(hydroxy)methyl]cinnamate,
(28) trans-4-[(5,5,8,8-tetramethyl-5,6,7,8-tetrahydro-2-naphthyl)(hydroxy)methyl]cinnamic acid,
(29) 4'-(2-hydroxyethyl)piperazino-4-[(5,5,8,8-tetramethyl-5,6,7,8-tetrahydro-2-naphthyl)carbonyl]-benzamide (sic),
(30) N-[3,5-bis(trifluoromethyl)-1-phenyl]-4-[(5,5,8,8-tetramethyl-5,6,7,8-tetrahydro-2-naphthyl)-carbonyl]benzamide,

16. Process for preparing the compounds according to any one of Claims 1 to 15, characterized in that it consists in reacting, in an organic solvent medium, a halide such as an acide chloride corresponding to the following formula:

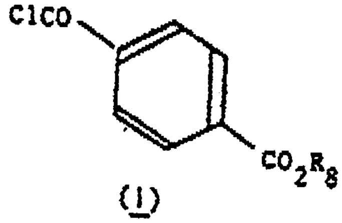

(1)

with an aromatic compound corresponding to one of the following formulae:

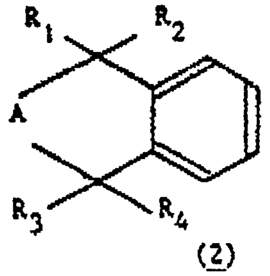

(2)

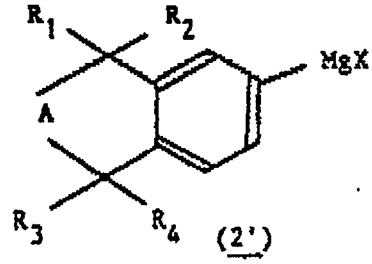

(2')

in which compounds:

A and $R_1$ to $R_4$ have the same meanings as those given in Claim 1, $R_8$ being an alkyl radical having from 1 to 20 carbon atoms, and X is Br or Cl, and in that this reaction is followed, if necessary, by the saponification of the keto ester obtained to the corresponding keto acid and by the subsequent conversion of the said keto acid to the corresponding amide by the action of an amine of

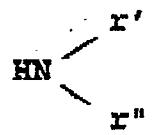

in which:

r' and r" have the same meanings as those given in Claim 1, or by the subsequent conversion of the said keto acid to a hydroxy acid or to a diol and by the oxidation, where appropriate, of the diol to the corresponding keto aldehyde.

17. Process according to Claim 16, characterized in that the condensation reaction with the aromatic compound (2) is performed in the presence of anhydrous aluminium chloride in 1,2-dichloroethane at a temperature of between 0 and 25°C with stirring.

18. Process according to Claim 16, characterized in that the condensation of the acid chloride with the organomagnesium compound (2) is performed in THF at a temperature of approximately 0°C.

19. Process according to Claim 16, characterized in that the preparation of the amide is performed in the presence of N,N'-carbonyldiimidazole.

20. Process according to Claim 16, characterized in that the reduction of the keto acid to the corresponding hydroxy acid is performed in the presence of sodium borohydride in THF.

21. Process according to Claim 16, characterized in that the keto aldehyde is obtained by oxidation of the diol using pyridinium chlorochromate, the said corresponding diol resulting from a reduction reaction of the keto acid in the presence of lithium aluminium hydride.

22. Process for preparing a compound of formula (I) in which n = O and R = CHO, characterized in that it consists in reacting, in an organic solvent medium, under the conditions of the Friedl-Crafts reaction, an acid chloride of the following formula:

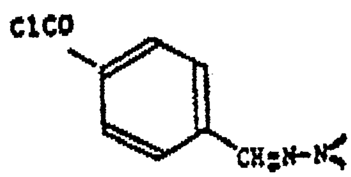

with an aromatic compound of formula:

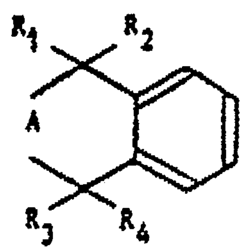

and in that this reaction is then followed by liberation of theh aldehyde group by exchange with glyoxal to lead to the expected keto aldehyde.

23. Process for preparing the compounds of formula:

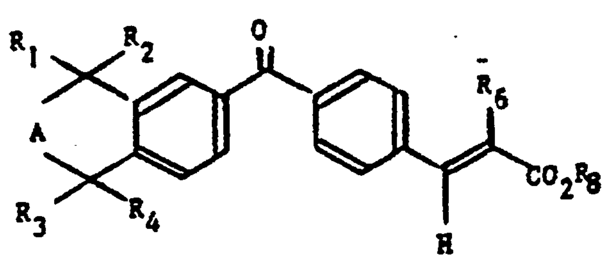

in which:

$R_1$ to $R_4$, $R_6$ and $R_8$ have the same meanings as in Claim 1,
characterized in that a keto aldehyde as obtained in Claim 16 or 22, and having the following formula:

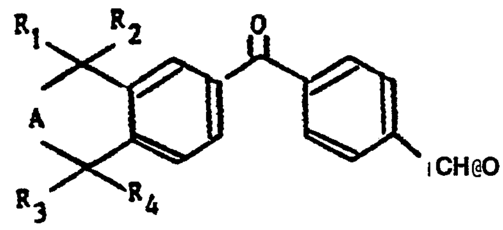

in which
A and $R_1$ to $R_4$ have the same meanings as in Claim 1, is reacted with an alkyl phosphonoacetate of formula:

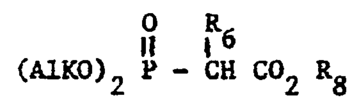

in the presence of sodium hydride in THF, and in that the unsaturated keto ester obtained is subjected to the conventional reaction conditions enabling access to be gained to the different meanings of the radicals of the formula (I) according to Claim 1.

24. Medicinal product, characterized in that it is a compound of formula (I) according to any one of Claims 1 to 15, or obtained according to any one of Claims 16 to 23.

25. Medicinal product according to Claim 24, characterized in that it is administered at a daily dose of approximately 2 µg/kg to 2 mg/kg of bodyweight.

26. Pharmaceutical composition, characterized in that it contains, in a vehicle suitable for enteral, parenteral, topical or ocular administration, at least one compound of formula (I) according to any one of Claims 1 to 15, or obtained according to any one of claims 16 to 23.

27. Composition according to Claim 26, characterized in that it is presented ina form suitable for topical or ocular administration, and contains from 0.0005 to approximately 5% by weight of a compound of formula (I).

28. Use of a compound according to any one of Claims 1 to 15, or obtained according to any one of Claims 16 to 23, for the preparation of a pharmaceutical composition intended for the treatment of dermatological, respiratory and also ophthalmological conditions.

29. Cosmetic composition for body and hair hygiene, characterized in that it contains, in a suitable cosmetic vehicle, at least one compound of formula (I) according to any one of Claims 1 to 15, or obtained according to any one of Claims 16 to 23.

30 Cosmetic composition according to Claim 29, characterized in that it contains the compound of formula (I) at a concentration of between 0.0005 and 2%, and preferably between 0.1 and 1%, by weight.

31. Composition according to either of Claims 26 and 29, characterized in that it contains, in addition, at least one inert additive or one pharmacodynamically or cosmetically active additive such as a moisturizing agent, an antiseborrhoeic agent, an anti-acne agent, an antibiotic, an agent promoting hair regrowth, an antiinflammatory agent, a carotenoid, an antipsoriatic agent, a flavour modifier, a preservative, a stabilizer, a moisture regulator, a pH regulator, an osmotic pressure-modifying agent, an emulsifier, a UV-A or UV-B screening agent or an antioxidant.

**Claims for the contracting states: AT, ES, GR**

1. Process for preparing an aromatic bicyclic compound corresponding to the following general formula:

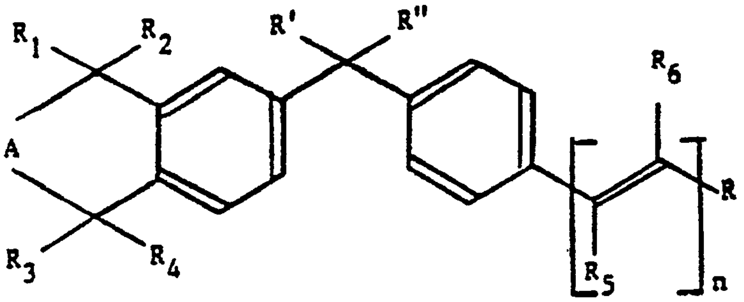

(I)

in which
n is 0 or 1,
R′ represents a hydrogen atom, an OH radical, an alkoxy radical having from 1 to 4 carbon atoms or an acyloxy radical having from 1 to 4 carbon atoms,
R″ represents a hydrogen atom or an alkoxy radical having from 1 to 4 carbon atoms,
or R′ and R″, taken together, form an oxo (=O), methano (=$CH_2$) or hydroxyimino (=N–OH) radical,
R represents a $-CH_2OH$ radical or a radical $-COR_7$,
$R_7$ representing a hydrogen atom, a radical $-OR_8$
or a radical

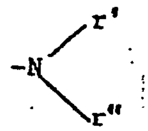

$R_8$ representing a hydrogen atom, an alkyl radical having from 1 to 20 carbon atoms, a monohydroxyalkyl, polyhydroxyalkyl or optionally substituted aryl or aralkyl radical or a sugar residue, or alternatively a radical

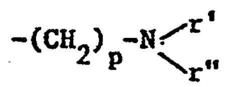

p being 1, 2 or 3 and r′ and r″ representing a hydrogen atom, a lower alkyl radical, a monohydroxyalkyl radical optionally interrupted by a hetero atom, a polyhydroxyalkyl radical, an optionally substituted aryl or benzyl radical, an amino acid residue or an amino sugar residue or, taken together with the nitrogen atom to which they are attached form a heterocycle,
A represents a methylene or dimethylene radical, unsubstituted or substituted with a lower alkyl radical
$R_1$, $R_2$, $R_3$ and $R_4$ represent a hydrogen atom or a lower alkyl radical,
it being possible for $R_1$ and $R_3$, taken together, to form a methylene or dimethylene radical when A represents a dimethylene radical, and
$R_5$ and $R_6$ represent a hydrogen atom or a methyl radical,
characterized in that it consists in reacting, in an organic solvent medium, a halide such as an acid chloride corresponding to the following formula:

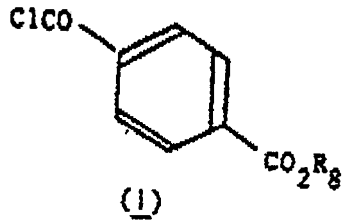

(1)

with an aromatic compound corresponding to one of the following formulae:

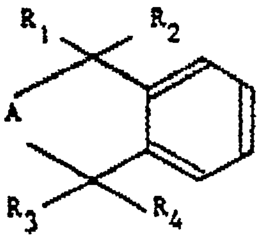

(2)

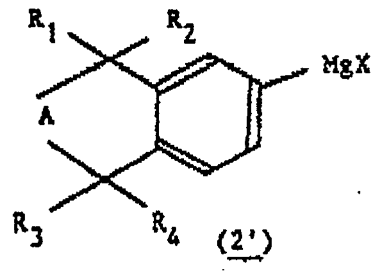

(2')

in which compounds:
A and $R_1$ to $R_4$ have the same meanings as those given above, $R_8$ being an alkyl radical having from 1 to 20 carbon atoms, and X is Br or Cl, and in that this reaction is followed, if necessary, by the saponification of the keto ester obtained to the corresponding keto acid and by the subsequent conversion of the said keto acid to the corresponding amide by the action of an amine of formula:

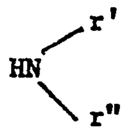

in which:
r′ and r″ have the same meanings as those given above or by the subsequent conversion of the said keto acid to a hydroxy acid or to a diol and by the oxidation, where appropriate, of the diol to the corresponding keto aldehyde.

2. Process according to Claim 1, characterized in that the condensation reaction with the aromatic compound (2) is performed in the presence of anhydrous aluminium chloride in 1,2-dichloroethane at a temperature of between 0 and 25°C wtih stirring.

3. Process according to Claim 1, characterized in that the condensation of the acid chloride with the organomagnesium compound (2) is performed in THF at a temperature of approximately 0°C.

4. Process according to Claim 1, characterized in that the preparation of the amide is performed in the presence of N,N′-carbonyldiimidazole.

5. Process according to Claim 1, characterized in that the reduction of the keto acid to the corresponding hydroxy acid is performed in the presence of sodium borohydride in THF.

6. Process according to Claim 1, characterized in that the keto aldehyde is obtained by oxidation of the diol using pyridinium chlorochromate, the said corresponding diol resulting from a reduction reaction of the keto acid in the presence of lithium aluminium hydride.

7. Process for preparing a compound of formula:

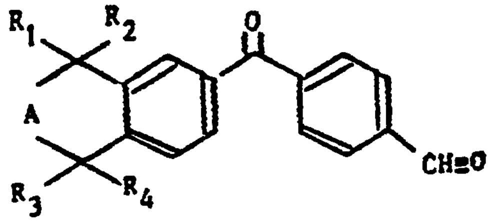

characterized in that it consists in reacting, in an organic solvent medium, under the conditions of the Friedel-Crafts reaction, an acid chloride of the following formula:

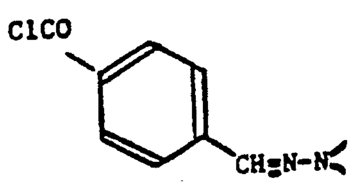

with an aromatic compound of formula:

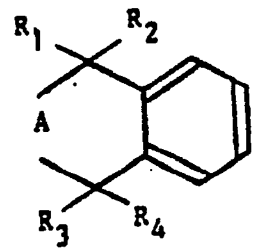

and in that this reaction is then followed by liberation of the aldehyde group by exchange with glyoxal lead to the expected keto aldehyde.

8. Process for preparing a compound of formula:

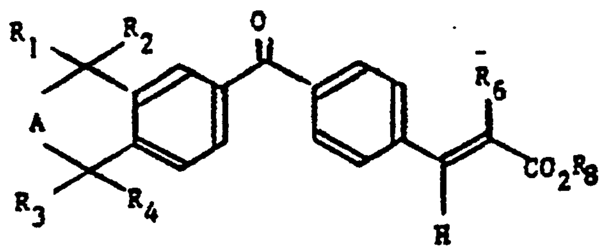

in which:
$R_1$ to $R_4$, $R_5$ and $R_8$ have the same meanings as in Claim 1,
characterized in that a keto aldehyde as obtained in Claims 1 to 7, and having the following formula:

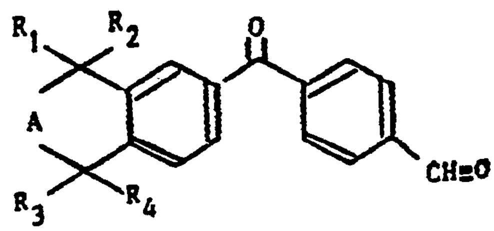

in which
A and $R_1$ to $R_4$ have the same meanings as in Claim 1, is reacted with an alkyl phosphonoacetate of formula:

$$(AlKO)_2\overset{O}{\overset{\|}{P}} - \overset{R_6}{\overset{|}{CH}}\ CO_2\ R_8$$

in the presence of sodium hydride in THF, and in that the unsaturated keto ester obtained is subjected to the conventional reaction conditions enabling access to be gained to the different meanings of the radicals of the formula (I) according to Claim 1.

9. Use of a compound obtained according to any one of Claims 1 to 8 for the preparation of a pharmaceutical composition intended for the treatment of dermatological, respiratory and also ophthalmological conditions.